(19) Europäisches Patentamt
European Patent Office
Office européen des brevets

(11) **EP 4 360 667 A1**

(12) **EUROPEAN PATENT APPLICATION**

(43) Date of publication:
**01.05.2024 Bulletin 2024/18**

(21) Application number: **22203338.3**

(22) Date of filing: **24.10.2022**

(51) International Patent Classification (IPC):
**A61M 1/34** (2006.01)    **A61M 1/36** (2006.01)

(52) Cooperative Patent Classification (CPC):
**A61M 1/3672; A61M 1/3434; A61M 1/3458;**
**A61M 1/361;** A61M 1/1643; A61M 2205/18

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Gambro Lundia AB**
**226 43 Lund (SE)**

(72) Inventor: **POUCHOULIN, Dominique**
**01390 Tramoyes (FR)**

(74) Representative: **PGA S.p.A., Milano, Succursale di Lugano**
**Via Castagnola, 21c**
**6900 Lugano (CH)**

(54) **EXTRACORPOREAL BLOOD TREATMENT APPARATUS**

(57)    An extracorporeal blood treatment apparatus 1 has a filtration unit 2, a blood circuit 6, and a main infusion line 9 connected to a blood withdrawal line 6a to infuse a solution into blood. A main infusion pump 11 causes the infusion solution I to flow from an infusion fluid source 10 to the blood withdrawal line, and an inactivated condition, wherein the main infusion pump 11 is inactive; a concentrate sensor 12 is placed on the blood withdrawal line between an injection point 9a and the filtration unit 2 for monitoring the concentration of a substance. A control unit 13, in the first operating condition, activates the main infusion pump 11 and acquires the signal from the concentrate sensor 12 to determine the concentration of the substance into blood infused with the infusion solution, and, in the second operating condition, inactivates the main infusion pump 11 and acquires the signal to determine the concentration of the substance when no infusion solution I is infused into the blood.

FIG.2

EP 4 360 667 A1

**Description**

BACKGROUND

The present disclosure relates generally to medical fluid treatments and in particular to extracorporeal blood treatments, such as dialysis fluid treatments.

Due to various causes, a person's renal system can fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with dialysis. Dialysis removes waste, toxins and excess water from the body that normal functioning kidneys would otherwise remove. Dialysis treatment for replacement of kidney functions is critical to many people because the treatment is lifesaving.

One type of kidney failure therapy is Hemodialysis ("HD"), which in general uses diffusion to remove waste products from a patient's blood. A diffusive gradient occurs across the semipermeable dialyzer between the blood and an electrolyte solution called dialysate or dialysis fluid to cause diffusion. HD fluids are typically created by the dialysis machines by mixing concentrates and clean water.

Hemofiltration ("HF") is an alternative renal replacement therapy that relies on a convective transport of toxins from the patient's blood. HF is accomplished by adding substitution or replacement fluid to the extracorporeal circuit during treatment. The substitution fluid and the fluid accumulated by the patient in between treatments is ultrafiltered over the course of the HF treatment, providing a convective transport mechanism that is particularly beneficial in removing middle and large molecules.

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is delivered directly to the extracorporeal circuit, providing convective clearance. Here, more fluid than the patient's excess fluid is removed from the extracorporeal blood of the patient, causing the increased convective transport of waste products from the patient. The additional fluid removed is replaced via the substitution or replacement fluid.

The apparatus of the present invention may also be used in the context of continuous renal replacement therapies (CRRT) with or without anticoagulation, for example CRRT with or without systemic anticoagulation (e.g., heparin) / with or without regional anticoagulation (e.g., citrate).

In particular, the apparatus of the present invention may be used in CRRT therapies and/or ECCO$_2$R, via extracorporeal blood circulation as an alternative or supplement to mechanical ventilation, and/or for monitoring regional citrate anticoagulation (RCA) during continuous renal replacement therapies (CRRT).

Irrespective of the type of therapy and/or treatment referred to, in-line sensing technology plays an important role in the efficient functioning of the extracorporeal blood treatment apparatuses.

It is known that some pieces of prior art describe use of in-line sensors for citrate anticoagulation monitoring, involving the presence of sensors upstream and/or downstream of the dialyzer/filter for the adjustment of citrate and/or calcium infusion rate.

US2011208105 provides different embodiments of a device for citrate anticoagulation- in an extracorporeal blood purification device and a method for detecting an ion concentration of bivalent cations of the blood during a citrate anti-coagulated extracorporeal blood purification.

A first embodiment comprises an extracorporeal blood purification system having an extracorporeal blood circuit, which comprises a dialysis unit, a blood inflow, a blood return, a citrate metering device for supplying citrate at a citrate supply point upstream the dialysis unit, a substitution medium metering device for supplying a substitution medium at a substitution medium supply point downstream the dialysis unit, at least one ion concentration measuring means for measuring bivalent cations, a controller adapted to regulate the metering of the substitution medium as a function of a comparison between a set point value range and the ion concentration measured through the ion concentration measuring means. The ion concentration measuring means is adapted for the continuous generation of measuring values and is arranged upstream the citrate supply point. The controller is adapted to continuously carry out a regulation of the metering of the citrate and of the substitution medium in consideration of a target value or target value range, which can be predetermined in the controller downstream the citrate supply point.

The concentration measuring means arranged upstream is a Ca-ion sensor through which the Ca-ion concentration is measured sensitively and specifically, being only able to measure the Ca-ion value of the patient's systemic blood. The adjustment of the citrate supply rate is based on a mathematical model. The Ca-ion concentration measured by the Ca-ion sensor represents the current intra-corporeal Ca-ion level and thus the physiological state of the patient.

Unlike the first embodiment, the second embodiment provides additional means for detecting Ca-ion values, i.e.:

- a first Ca-Ion concentration sensor arranged in the extracorporeal blood circuit between the citrate supply point and the dialysis unit for measuring the Ca-ion concentration of the anti-coagulated blood prior to the entry into the dialysis unit, in response to an effective anti-coagulation;

- a second Ca-ion sensor that can furthermore be arranged between the dialysis unit and the substitution medium supply point for measuring the Ca-ion concentration after the dialysis and thus providing for the accurate metering/control of the substitution medium;
- a third Ca-ion sensor that can be arranged in the closed plasma circuit; in the case of blood purification systems comprising a plasma circuit, in the case of which a measuring of the Ca-ion concentration is provided between the citrate supply point and the dialysis filter, the measuring can take place through the third Ca-ion sensor in addition to or instead of a measuring through the first Ca-ion sensor;
- a fourth Ca-ion sensor that can be arranged downstream the substitution medium supply point regulating the substitution medium supply rate;
- a fifth and a sixth Ca-ion sensors that can be arranged upstream the citrate supply point; the Ca-ion concentration is measured alternately by the fifth and the sixth Ca-ion sensors, wherein one of such sensors is calibrated, while the other generates a measuring signal; one of such Ca-ion sensors can detect the Ca-ion concentration, whereas and the other one can detect the Mg-ion concentration.

The measured values obtained by the first and the third Ca-ion sensors allow the controller to regulate the citrate supply rate against the pre-adjusted target value.

DE 10114 283 C2 discloses a method for determining the concentration of an ion, atom, or molecule bound in a complex. In particular, it refers to a hemodialysis method with citrate anticoagulation. By adding trisodium citrate to the part of the extracorporeal circuit leading from the patient to the dialyzer, the concentration of free Ca-ions is reduced to such an extent that the coagulation cascade is interrupted. A tricalcium bicitrate complex is formed. A Ca-free dialysate prevents any transfer of calcium ions from the dialysis fluid towards the blood.

In order to compensate for the removal of calcium and magnesium, which is also bound by citrate, occurring in the dialyzer, a substitution solution containing Ca and Mg ions in an adapted concentration is injected into the venous' line of the extracorporeal circuit or in a separate venous access.

To control the anticoagulation process, the formation of the Ca-citrate complex is prevented by temporarily interrupting the addition of citrate. The Ca concentration increases accordingly and the Ca-ion concentration may be measured on the dialysate side with the Ca sensor. The Ca ion concentration in the patient's blood may be determined by reducing the dialysate flow to such an extent that the Ca-ion concentration on the dialysate side is adjusted to the Ca-ion concentration on the blood side.

Without changing the usual treatment blood and dialysate flow, the blood-side concentration may also be determined from the measured dialysate-side concentration using the relationships between the blood-side input concentration of Ca-ions, the measured dialysate-side output concentration of Ca-ions, the dialysate flow and the clearance.

Alternatively, the method may be effected without interrupting the citrate infusion. In this case, the Ca-ion concentration is determined by the fact that the Ca-ion is released again from the Ca citrate complex. This is done by supplying an acid. As a result, the pH reduction leads to a dissociation of the complex and the Ca-ions are correspondingly released.

US2018303995 refers to an extracorporeal filtration and detoxification system and method including: separating ultrafiltrate from cellular components of blood; treating the ultrafiltrate independently of the cellular components in a recirculation circuit; recombining treated ultrafiltrate and the cellular components; returning whole blood to the patient. Several embodiments are shown and disclosed. In various embodiments, the system may include one or more calcium and/or citrate sensors disposed along the blood circuit to monitor ionized calcium and/or citrate concentrations. The sensors may be configured to detect levels of ionized calcium, citrate, and/or calcium citrate. In one specific embodiment, the system includes a sensor adjacent each citrate or calcium infusion port along the blood circuit. A calcium sensor may be located downstream a calcium infusion port and upstream of an active cartridge. Additionally, a calcium sensor may be located downstream the active cartridge and downstream a junction.

US2007007184 A1 refers to a hemodialysis system in which at least one disposable sensor is combined with a dialysis circuit, in combination with an optional transceiver, to provide real-time or near-real-time patient data. Sensors for any substance or sensors of calcium levels may be included in the blood circuit. Sensors may be positioned upstream dialyzer.

SUMMARY

The present disclosure sets forth extracorporeal blood treatment apparatus, used for performing extracorporeal blood treatments (e.g., dialysis), such as hemofiltration (HF), hemodialysis ("HD"), hemodiafiltration ("HDF"), hemoperfusion, toxins adsorption, as well as the treatments performed during continuous renal replacement treatments ("CRRT").

In light of the disclosure set forth herein, and without limiting the disclosure in any way, in a 1st independent aspect, which may be combined with any other aspect or portion thereof described herein, an extracorporeal blood treatment apparatus (1) comprises: a filtration unit (2); an extracorporeal blood circuit (6) comprising a blood withdrawal line (6a) connected to an inlet (3a) of the filtration unit (2), and a blood return line (6b) connected to an outlet (3b) of the filtration unit (2); at least one main infusion line (9) connected to the blood withdrawal line (6a) of the extracorporeal blood circuit (6) to transfer an infusion solution (I) into blood; at least one infusion fluid source (10) of the infusion solution (I) connected to the main infusion line (6a); a main infusion pump (11) active on the main infusion line (9) drivable between an activated condition wherein the main infusion pump (11) causes the infusion solution (I) to flow from the corresponding infusion fluid source (10) to the blood withdrawal line (6a) of the extracorporeal blood circuit (6), and an inactivated condition, wherein the main infusion pump (11) is inactive and the infusion solution (I) does not flow through the corresponding main infusion line (9); a concentrate sensor (12) placed on the blood withdrawal line (6a) between an injection point (9a) of the main infusion line (9) and the filtration unit (2) for monitoring the concentration of at least one substance detectable into blood flowing into the blood withdrawal line (6a) between the injection point (9a) and the filtration unit (2); a control unit (13) operatively connected to the concentration sensor (12) and the main infusion pump (11) of the main infusion line (9), the control unit (13) being configured to drive the main infusion pump (11) between the activated condition and the inactivated condition and to receive signals from the concentrate sensor (12) to determine the concentration of the at least one substance in a first and in a second operating condition of the extracorporeal blood treatment apparatus (1), wherein the control unit (13) is further configured to: (i) in the first operating condition - activate the main infusion pump (11) and acquire the signal from the concentrate sensor (12) to determine the concentration of the at least one substance into blood infused with the infusion solution (I); and, (ii) in the second operating condition - inactivate the main infusion pump (11) and acquire the signal from the concentrate sensor (12) to determine the concentration of the at least one substance into blood when no infusion solution (I) is infused into the blood.

In a 2nd independent aspect, which may be combined with any other aspect or portion thereof described herein, an extracorporeal blood treatment apparatus (1) comprises: a filtration unit (2); an extracorporeal blood circuit (6) comprising a blood withdrawal line (6a) connected to an inlet (3a) of the filtration unit (2), and a blood return line (6b) connected to an outlet (3b) of the filtration unit (2); at least one main infusion line (9) connected to the blood withdrawal line (6a) of the extracorporeal blood circuit (6) to transfer an infusion solution (I) into blood; at least one infusion fluid source (10) of the infusion solution (I) connected to the main infusion line (6a); a main infusion pump (11) active on the main infusion line (9) drivable between an activated condition, wherein the main infusion pump (11) causes the infusion solution (I) to flow from the corresponding infusion fluid source (10) to the blood withdrawal line (6a) of the extracorporeal blood circuit (6), and an inactivated condition, wherein the main infusion pump (11) is inactive and the infusion solution (I) does not flow through the corresponding main infusion line (9); a concentrate sensor (12) placed on the blood withdrawal line (6a) between an injection point (9a) of the main infusion line (9) and the filtration unit (2) for monitoring the concentration of at least one solute detectable into blood flowing into the blood withdrawal line (6a) between the injection point (9a) and the filtration unit (2); a control unit (13) operatively connected to the concentration sensor (12) and the main infusion pump (11) of the main infusion line (9), the control unit (13) being configured to drive the main infusion pump (11) between the activated condition and the inactivated condition and to receive signals from the concentrate sensor (12) to determine the concentration of the at least one solute, wherein the control unit (13) is further configured to run a concentrate computation routine comprising the following steps:

- acquire the concentration of the at least one solute in the infusion fluid source (10);
- acquire an infusion flow rate through the main infusion line (9); acquire a blood flow rate through the blood circuit (6);
- activate the main infusion pump (11); acquire the signal from the concentrate sensor (12) when blood is infused with the infusion solution (I);
- calculate a patient systemic concentration of the at least one solute based on the signal from the concentrate sensor (12), the concentration of the at least one solute in the infusion fluid source (10), the infusion flow rate and the blood flow rate,
- wherein the patient systemic concentration of the at least one solute is the at least one solute concentration in the blood withdrawal line (6a) upstream the injection point (9a) of the main infusion line (9).

In a 3rd independent method aspect which may be combined with any other aspect or portion thereof described herein, a monitoring method (MM) for monitoring at least one substance (S) into a fluid (F), in particular blood, flowing through an extracorporeal blood circuit (6) of an extracorporeal blood apparatus (1) of the type comprising a filtration unit (2), a blood pump (17) placed upstream the filtration unit (2) and a main infusion line (9) arranged to transfer an infusion solution (I) into the supplied fluid (F) upstream the filtration unit (2), comprises the following steps:

- (S1) supplying a fluid (F) to be monitored, in particular blood as withdrawn from a patient (P), to the filtration unit (2);
- (S2) infusing at least one infusion solution (I) into the fluid (F) supplied to the filtration unit (2), the infusion solution (I) being injected into the fluid (F) supplied to the filtration unit (2) at an injection point (9a) of the main infusion line (9) placed upstream the filtration unit (2);
- (S3) detecting the concentration of at least one substance into the fluid (F) infused with at least one of the infusion solution (I) downstream the injection point (9a) of the main infusion line (9) and upstream the filtration unit (2);

• (S4) interrupting the infusion of at least one of the infusion solution (I) into the fluid (F) supplied to the filtration unit (2) to allow a fluid (F) not infused with any infusion solution (I) to flow towards the filtration unit (2);

• (S5) detecting the concentration of at least one substance into the fluid (F) not infused with at least one of the infusion solution (I) downstream the injection point (9a) of the main infusion line (9) and upstream the filtration unit (2);

• (S6) optionally, repeating one or more steps of, or one or more sequences of the steps of (S1) to (S5).

Steps S1, S2, optionally S3, S4, and optionally S5 being executed by the control unit of the extracorporeal blood treatment apparatus 1.

In a 4<sup>th</sup> independent method aspect which may be combined with any other aspect or portion thereof described herein, a monitoring method (MM) for monitoring at least one substance (S) into a fluid (F), in particular blood, flowing through an extracorporeal blood circuit (6) of an extracorporeal blood apparatus (1) of the type comprising a filtration unit (2), a blood pump (17) placed upstream the filtration unit (2), a main infusion line (9) arranged to transfer an infusion solution (I) into the supplied fluid (F) upstream the filtration unit (2), an auxiliary infusion line (14) arranged to transfer an auxiliary infusion solution (II) into the supplied fluid (F) upstream the filtration unit (2), the method comprising the following steps:

• (P1) supplying a fluid (F) to be monitored, in particular blood as withdrawn from a patient (P), to the filtration unit (2);

• (P2) infusing at least one infusion solution (I) and/or at least one auxiliary infusion solution (II) into the fluid supplied to the filtration unit (2), the infusion solution (I) and/or the auxiliary infusion solution (II) being injected into the fluid (F) supplied to the filtration unit (2) at a corresponding injection point (9a, 14a) of the corresponding infusion line (9, 14) placed upstream the filtration unit (2);

• (P3) detecting the concentration of at least one substance into the fluid (F) infused with at least one of the infusion solutions (I, II) downstream the corresponding injection point (9a, 14a) of the corresponding infusion line (9, 14) and upstream the filtration unit (2);

• (P4) interrupting the infusion of at least one of the infusion solutions (I, II) into the fluid supplied to the filtration unit (2) or interrupting the infusion of both infusion solutions (I, II) into the fluid supplied to the filtration unit (2) to respectively allow:

  ○ a fluid (F) infused with only one infusion solution (I, II);
  ○ a fluid (F) not infused with any infusion solution (I, II),
  to flow towards the filtration unit (2);

• (P5) detecting the concentration of at least one substance into the fluid (F) infused with only one infusion solution (I, II) or not infused with any infusion solution (I, II) downstream the corresponding injection point (9a, 14a) of the corresponding infusion line (9, 15) and upstream the filtration unit (2):

• (P6) optionally, repeating one or more steps of, or one or more sequences of the steps of (P1) to (P5).

Steps P1, P2, optionally P3, P4, and optionally P5 being executed by the control unit of the extracorporeal blood treatment apparatus 1.

In another independent aspect, an extracorporeal blood treatment apparatus (1) is provided comprising: a filtration unit (2); an extracorporeal blood circuit (6) comprising a blood withdrawal line (6a) connected to an inlet (3a) of the filtration unit (2), and a blood return line (6b) connected to an outlet (3b) of the filtration unit (2); at least one main infusion line (9) connected to the blood withdrawal line (6a) of the extracorporeal blood circuit (6) to transfer an infusion solution (I) into blood; at least one infusion fluid source (10) of the infusion solution (I) connected to the main infusion line (6a); a main infusion pump (11) active on the main infusion line (9) drivable between an activated condition wherein the main infusion pump (11) causes the infusion solution (I) to flow from the corresponding infusion fluid source (10) to the blood withdrawal line (6a) of the extracorporeal blood circuit (6), and an inactivated condition, wherein the main infusion pump (11) is inactive and the infusion solution (I) does not flow through the corresponding main infusion line (9); a sensor coupling placed on the blood withdrawal line (6a) between an injection point (9a) of the main infusion line (9) and the filtration unit (2), the sensor coupling being configured to receive a concentration sensor (12) for monitoring the concentration of at least one substance detectable into blood flowing into the blood withdrawal line (6a) between the injection point (9a) and the filtration unit (2); a control unit (13) operatively connected to the main infusion pump (11) of the main infusion line (9), the control unit (13) being configured to drive the main infusion pump (11) between the activated condition and the inactivated condition and to receive a value of the concentration of the at least one substance in a first and in a second operating condition of the extracorporeal blood treatment apparatus (1), wherein the control unit (13) is further configured to:

in the first operating condition - activate the main infusion pump (11) to allow coupling the concentration sensor (12) for determining the concentration of the at least one substance into blood infused with the infusion solution (I); and,

in the second operating condition - inactivate the main infusion pump (11) and allow coupling the concentration sensor (12) for determining the concentration of the at least one substance into blood when no infusion solution (I) is infused into the blood.

In the previous independent aspects, a filtration unit 2 and a blood circuit connected to the filtration unit is included. In corresponding alternative independent aspects, the filtration unit may be absent and a gas exchanger 46 (or another blood treatment device) used instead of the filtration unit. In this case, the blood withdrawal line 6a is connected to an inlet of the gas exchanger 46 (or device), and a blood return line 6b is connected to an outlet of the gas exchanger 46 (or device). The control unit will be configured to execute the same procedure for the first and second operating conditions.

In the following aspects details are added with regard to both the extracorporeal blood treatment apparatus (1) and the methods considered in the previous independent aspects. Each and every aspect relating to the components/elements and/or any feature of each extracorporeal blood treatment apparatus/method considered in the previous independent aspects are clearly combinable with the aspects of the other extracorporeal blood treatment apparatus (1) considered both in the independent aspects and in the dependent aspects.

Furthermore, when the following aspects depends on one of the previous independent method aspects such as the 3rd or the 4th, or both of the previous independent aspects such as the 3rd and the 4th, any feature of the apparatus (1) is related to the corresponding method (MM) and/or can be considered and converted into corresponding method aspects.

In a 5th aspect according to any of previous aspects, the control unit (13) is configured to switch between first and second operating conditions (i), (ii) and to repeat such operating conditions (i) and (ii) a plurality of times during an extracorporeal blood treatment.

The first operating condition corresponds to the situation where the main infusion pump (11) is active and the signal from the concentrate sensor (12) is used to determine the concentration of the at least one substance into blood infused with the infusion solution (I). It includes steps S1, S2, and S3 of aspect 3 as well as steps P1, P2, and P3 of aspect 4. The second operating condition corresponding to a situation where the main infusion pump (11) is inactivated and the signal from the concentrate sensor (12) is used to determine the concentration of the at least one substance into blood when no infusion solution (I) is infused into the blood. It includes steps S4 and S5 of aspect 3 as well as steps P4, and P5 of aspect 4.

In a 6th aspect according to any of the previous aspects, the filtration unit (2) has a primary chamber (3) and a secondary chamber (4) separated by a semi-permeable membrane (5), the blood withdrawal line (6a) being connected to the inlet (3a) of the primary chamber (3) of the filtration unit (2), the blood return line (6b) being connected to the outlet (3b) of the primary chamber (3) of the filtration unit (2).

In a 7th aspect according to any of the previous aspects, the filtration unit (2) has a primary chamber (3) and a secondary chamber (4) separated by a semi-permeable membrane (5), the apparatus comprising a dialysate line (7) connected to an outlet (4b) of the secondary chamber (4) and optionally a supply line (8) connected to an inlet (4a) of the secondary chamber (4).

In an 8th aspect according to any one of the previous aspects, the second operating condition (ii), the control unit (13) is configured to keep the main infusion pump (11) in the inactivated condition to allow blood with no infusion solution (I) infused by the main infusion line (9) to reach the concentration sensor (12), the control unit (13) detecting the concentration of the at least one substance into blood when blood with no infusion solution (I) reaches the concentration sensor (12).

In a 9th aspect according to any one of the previous aspects, in the second operating condition (ii), the control unit (13) is configured to keep the main infusion pump (11) in the inactivated condition for a predetermined time period, optionally between 10 sec and 300 sec with a blood flow rate in a range between 20 and 300 ml/min, in particular 50 and 300 ml/min.

In a 10th aspect according to any one of the previous aspects, in the second operating condition (ii), the control unit (13) is configured to keep the main infusion pump (11) in the inactivated condition for a time period which is at least a function of the blood flow rate in the blood withdrawal line (6a) of the extracorporeal blood circuit (6), in particular the blood flow rate in correspondence of the concentrate sensor (12).

In a 11th aspect according to any one of the previous aspects, in the second operating condition (ii), the control unit (13) is configured to keep the main infusion pump (11) in the inactivated condition for a time period which is at least a function of the cross section of the withdrawal line (6a) of the extracorporeal blood circuit (6), in particular in correspondence of the concentrate sensor (12).

In a 12th aspect according to any one of the previous aspects, in the second operating condition (ii), the control unit (13) is configured to keep the main infusion pump (11) in the inactivated condition for a time period which is at least a function of a distance on the withdrawal line (6a) of the extracorporeal blood circuit (6) between the injection point (9a) of the main infusion line (9) and the concentrate sensor (12).

In a 13th aspect according to any one of the previous aspects, in the second operating condition (ii), the control unit (13) is configured to keep the main infusion pump (11) in the inactivated condition for a time period which is at least a function of the design of a portion of the withdrawal line (6a) of the extracorporeal blood circuit (6) between the injection point (9a) of the main infusion line (9) and the concentrate sensor (12).

In a 14th aspect according to any one of the previous aspects, in the second operating condition (ii), the control unit (13) is configured to keep the main infusion pump (11) in the inactivated condition for a time period which is at least a function of a volume of a portion of the withdrawal line (6a) of the extracorporeal blood circuit (6) between the injection point (9a) of the main infusion line (9) and the concentrate sensor (12).

In a 15th aspect according to any one of the previous aspects, in the second operating condition (ii), the control unit (13) is configured to keep the main infusion pump (11) in the inactivated condition for a time period (t) function of the blood flow rate ($Q_b$) and the volume (V) of a circuit section between the infusion site (9a) and the sensor (12) according to the following relation:

$$t = \alpha \cdot \frac{V}{Q_b} + T_{rep}$$

wherein:

    t = time period;
    a = constant
    V = volume of the circuit section between the infusion site (9a) and the sensor (12);
    $Q_b$ = blood flow rate;

$T_{rep}$ = sensor response time. The higher the blood flow rate ($Q_b$) the lower is the time to 'rinse' the circuit section between infusion site (9a) and sensor (12) location. Typically, concentration becomes stable having flowed 3 times the volume of this circuit section. This means that constant $\alpha$ assumes a value of 3. Typical time range is between 10 s and 30 s.

Sensor response time ($T_{rep}$) is dependent on the sensor technology. Range may vary, for example between 30 s and 300 s. Notably, in the case the sensing device is not a true 'in-line sensor' but integrates a blood sampling function, sensor time response ($T_{rep}$) becomes zero (even if the actual measurement value becomes available after some processing time of the sample).

In a 16th aspect according to any one of the previous aspects, in the first operating condition (i), the control unit (13) is configured to keep the main infusion pump (11) in the activated condition to allow the concentration sensor (12) to detect the at least one substance into blood infused with the infusion solution (I) supplied by the infusion fluid source (10) through the main infusion line (9).

In a 17th aspect according to any one of the previous aspects, in the first operating condition (i), the control unit (13) is configured to keep the main infusion pump (11) in the activated condition for substantially the entire treatment except:

-    when concentration of the at least one substance into blood when no infusion solution (I) is infused is requested; and/or

-    when the infusion fluid source (10) of the infusion solution (I) is to be changed, in particular concentration of the at least one substance into blood when no infusion solution (I) is infused being measured at least during infusion fluid source (10) change.

In a 18th aspect according to any one of the previous aspects, in the first operating condition (i), the control unit (13) is configured to acquire the signal from the concentrate sensor (12) to determine the concentration of the at least one substance into blood infused with the infusion solution (I) after the main infusion pump (11) is in the activated condition for a time period which is at least a function of the blood flow rate on the blood withdrawal line (6a) of the extracorporeal blood circuit (6), in particular the blood flow rate in correspondence of the concentrate sensor (12).

In a 18th bis aspect according to any one of the previous aspects, in the first operating condition (i), control unit (13) is configured to acquire the signal from the concentrate sensor (12) to determine the concentration of the at least one substance into blood infused with the infusion solution (I) after the main infusion pump (11) is in the activated condition for a time period (t) function of the blood flow rate ($Q_b$) and the volume (V) of a circuit section between the infusion site (9a) and the sensor (12) in particular according to the following relation:

$$t = \alpha \cdot \frac{V}{Q_b} + T_{rep}$$

wherein:

    t = time period;
    a = constant
    V = volume of the circuit section between the infusion site (9a) and the sensor (12);
    $Q_b$ = blood flow rate;

$T_{rep}$ = sensor response time. The higher the blood flow rate ($Q_b$) the lower is the time to 'rinse' the circuit section between infusion site (9a) and sensor (12) location. Typically, concentration becomes stable having flowed 3 times the volume of this circuit section. This means that constant $\alpha$ assumes a value of 3. Typical time range is between 10 s and 30 s.

Sensor response time ($T_{rep}$) is dependent on the sensor technology. Range may vary, for example between 30 s and 300 s. Notably, in the case the sensing device is not a true 'in-line sensor' but integrates a blood sampling function, sensor time response ($T_{rep}$) becomes zero (even if the actual measurement value becomes available after some processing time of the sample).

In a 19th aspect according to any one of the previous aspects, in the first operating condition (i), the control unit (13) is configured to acquire the signal from the concentrate sensor (12) to determine the concentration of the at least one substance into blood infused with the infusion solution (I) after the main infusion pump (11) is in the activated condition for a time period which is at least a function of the cross section of the blood withdrawal line (6a) of the extracorporeal blood circuit (6), in particular the blood flow rate in correspondence of the concentrate sensor (12).

In a 20th aspect according to any one of the previous aspects, in the first operating condition (i), the control unit (13) is configured to acquire the signal from the concentrate sensor (12) to determine the concentration of the at least one substance into blood infused with the infusion solution (I) after the main infusion pump (11) is in the activated condition for a time period which is at least a function of a distance on a portion of the blood withdrawal line (6a) of the extracorporeal blood circuit (6) between the injection point (9a) of the main infusion line (9) and the concentrate sensor (12).

In a 21st aspect according to any one of the previous aspects, in the first operating condition (i), the control unit (13) is configured to acquire the signal from the concentrate sensor (12) to determine the concentration of the at least one substance into blood infused with the infusion solution (I) after the main infusion pump (11) in the activated condition for a time period which is at least a function of the design of a portion of the withdrawal line (6a) of the extracorporeal blood circuit (6) between the injection point (9a) of the main infusion line (9) and the concentrate sensor (12).

In a 22nd aspect according to any one of the previous aspects, in the first operating condition (i), the control unit (13) is configured to acquire the signal from the concentrate sensor (12) to determine the concentration of the at least one substance into blood infused with the infusion solution (I) after the main infusion pump (11) is in the activated condition for a time period which is at least a function of a volume of a portion of withdrawal line (6a) of the extracorporeal blood circuit (6) between the concentrate sensor (12) and the injection point (9a) of the main infusion line (9).

In a 23rd aspect according to any one of the previous aspects, in the first operating condition (i), the control unit (13) is configured to acquire the signal from the concentrate sensor (12) to determine the concentration of the at least one substance into blood infused with the infusion solution (I) after the main infusion pump (11) is in the activated condition for a time period which allows the concentrate sensor (12) to read a stable concentration value.

In a 24th aspect according to any one of the previous aspects, the at least one substance the concentrate sensor (12) is configured to detect into blood or blood infused with the infusion solution (I) supplied by the infusion fluid source (10) is a blood electrolyte such as sodium, potassium, phosphates, magnesium, chloride. The possibility to measure an electrolyte in its systemic concentration (i.e., when the infusion of fluid is stopped) allows both to check the patient actual systemic value during the treatment and also to check whether any 'calculated' or 'estimated' systemic value of the concentration of the same electrolyte (by a mathematical model for example) is correct or should be corrected.

In a 25th aspect according to any one of the previous aspects, the at least one substance the concentrate sensor (12) is configured to detect into blood or blood infused with the infusion solution (I) supplied by the infusion fluid source (10) is at least ionized calcium.

In a 26th aspect according to any one of the previous aspects, the concentrate sensor (12) is configured to detect the concentration of at least two different substances into blood or blood infused with the infusion solution (I) supplied by the infusion fluid source (10), in particular one of the two different substances being ionized calcium.

In a 27th aspect according to any one of the previous aspects, the concentrate sensor (12) is configured to separately detect the concentration of two different substances in sequence both into blood and into blood infused with the infusion solution (I) supplied by the infusion fluid source (10).

In a 28th aspect according to any one of the previous aspects, the concentrate sensor (12) is configured to simultaneously detect the concentration of two different substances into blood or blood infused with the infusion solution (I) supplied by the infusion fluid source (10).

In a 29th aspect according to any one of the previous aspects, the concentrate sensor (12) comprises an optical concentrate sensor, such as a fluorescence optical concentrate sensor.

In a 30th aspect according to any one of the previous aspects, the concentrate sensor (12)) involves micro sampling.

In a 31st aspect according to any one of the previous aspects, the concentrate sensor (12) is configured to detect concentration of a substance that is not present in the infusion solution (I).

In a 32nd aspect according to any one of the previous aspects, the infusion solution (I) supplied by the infusion fluid source (10) includes at least one solute that reacts with at least one component present in the blood, such as citrate reacting with ionized calcium that is present in the blood, in particular the concentrate sensor being configured to sense concentration of said component present in the blood (e.g., ionized calcium).

In a 33rd aspect according to any one of the previous aspects, the infusion solution (I) supplied by the infusion fluid source (10) includes at least one regional anticoagulant, in particular citrate and/or citric acid.

In a 34th aspect according to any one of the previous aspects, the control unit (13) is configured to adjust the amount of the infusion solution (I) supplied by the infusion fluid source (10) to be infused into the withdrawal line (6a) on the base of the concentration of the at least one substance detected by the concentrate sensor (12), in particular based on the concentration measured during the first operating condition (i).

In a 35th aspect according to any one of the previous aspects, the extracorporeal blood treatment apparatus (1) comprises only one concentrate sensor (12) active on the extracorporeal blood circuit (6) (i.e., no other concentrate sensors being located on the blood circuit), configured to detect at least one substance into blood or blood infused with the infusion solution (I) supplied by the infusion fluid source (10), in particular configured to detect at least ionized calcium into blood or blood infused with the infusion solution (I) supplied by the infusion fluid source (10).

In a 36th aspect according to any one of the previous aspects, the extracorporeal blood treatment apparatus (1) does not comprise other ion concentrate sensors except the one active on the blood withdrawal line (6a) of the extracorporeal blood circuit (6) (i.e., no other concentrate sensors being located on the blood circuit and on the fluid circuit) configured to detect at least one substance into blood or blood infused with the infusion solution (I) supplied by the infusion fluid source (10), in particular configured to detect at least ionized calcium into blood or blood infused with the infusion solution (I) supplied by the infusion fluid source (10).

In a 37th aspect according to any one of the previous aspects, the infusion fluid source (10) comprises at least one bag, optionally made of a plastic material.

In a 39th aspect according to any one of the previous aspects, the bag of the infusion fluid source (10) is flexible and made of two opposite leaves joined, optionally hermetically sealed, each other, in particular welded each other.

In a 40th aspect according to any one of the previous aspects, the bag of the infusion fluid source (10) is disposable.

In a 41st aspect according to any one of the previous aspects, the extracorporeal blood treatment apparatus (1) comprises a blood pump (17) active on the extracorporeal blood circuit (6).

In a 42nd aspect according to any one of the previous aspects, the blood pump (17) is placed on the withdrawal line (6a) of the extracorporeal blood circuit (6).

In a 43rd aspect according to any one of the previous aspects, the blood pump (17) is placed between the injection point (9a) of the main infusion line (9) and the filtration unit (2).

In a 44th aspect according to any one of the previous aspects, the blood pump (17) is placed between the concentrate sensor (12) and the filtration unit (2).

In a 44th bis aspect according to any one of the previous aspects 1 to 43, the blood pump (17) is placed downstream the concentrate sensor (12) and the injection point (9a) of the main infusion line (9).

In a 45th aspect according to any one of the previous aspects, the extracorporeal blood treatment apparatus further comprises an ion replenishment infusion line (19), an ion replenishment infusion pump (20) active on the ion replenishment infusion line (19) and a source (21) of ion replenishment solution (III) connected to one end of the ion replenishment infusion line (19), the ion replenishment infusion line (19) infusing the ion replenishment solution (19) either into the blood return line (6b) or directly into the patient (P), in particular the ion replenishment solution (III) comprising ionized calcium, specifically a concentrated solution of ionized calcium.

In a 46th aspect according to the previous aspect, the control unit (13) is configured to adjust the amount of the ion replenishment solution (III) supplied by the source (21) of ion replenishment solution (III) on the base of the concentration of the at least one substance detected by the concentrate sensor (12), in particular based on the concentration measured during the second operating condition (ii), such as the patient systemic concentration of ionized calcium measured during the second operating condition (ii).

In a 47th aspect according to any one of the previous aspects, the extracorporeal blood treatment apparatus (1) comprises a user interface comprising a screen for displaying data pertaining to the extracorporeal blood treatment apparatus (1), the measured concentrations of the at least one substance in the first (i) and in the second operating condition (ii) of the extracorporeal blood treatment apparatus (1) being displayed on the screen, in particular versus one or more different substances being monitored, optionally the measured concentrations of the at least one substance in the first operating condition (i) being displayed as a chart over time and/or the measured concentrations of the at least one substance in the second operating condition (ii) being displayed as a chart over time.

In a 47th bis aspect according to any one of the previous aspects, the extracorporeal blood treatment apparatus (1) comprises a user interface comprising a screen for displaying data pertaining to the extracorporeal blood treatment apparatus (1), the control unit (13) being configured to display the measured concentrations of the substance (particularly measured during the first operating condition), for example ionized calcium, versus anticoagulant dose, for example citrate dose.

In a 48th aspect according to any one of the previous aspects, the control unit (13) is configured to compare the measured concentration of the at least one substance in the first operating condition (i) with a first high threshold and to issue an alarm in case the measured concentration exceeds the first high threshold, in particular the alarm being issued if ionized calcium concentration is over the first high threshold, such as ionized calcium higher than 0.40 mM.

In a 48th bis aspect according to any one of the previous aspects, the control unit (13) is configured to compare the measured concentration of the at least one substance in the first operating condition (i) with a first low threshold and to issue an alarm in case the measured concentration exceeds the first low threshold, in particular the alarm being issued if ionized calcium concentration is below the first low threshold, such as ionized calcium lower than 0.25 mM.

In a 49th aspect according to any one of the previous aspects, the control unit (13) is configured to compare the measured concentration of the at least one substance in the second operating condition (ii) with a second low threshold and to issue an alarm in case the measured concentration exceeds the second low threshold, in particular the alarm being issued if ionized calcium concentration is below the second low threshold, such as ionized calcium lower than 0.90 mM.

In an aspect 49th bis according to any one of the previous aspects, the control unit (13) is configured to compare the measured concentration of the at least one substance in the second operating condition (ii) with a second high threshold and to issue an alarm in case the measured concentration exceeds the second high threshold, in particular the alarm being issued if ionized calcium concentration is beyond the second high threshold, such as ionized calcium higher than 1.20 mM.

In a 50th aspect according to any one of the previous aspects, the control unit (13) is configured to determine a variation of the measured concentration of the at least one substance in the second operating condition (ii) and to compare the variation with a drift threshold and to issue an alarm in case the variation exceeds the drift threshold, in particular the alarm being issued if ionized calcium concentration is varying faster than the drift threshold, such as ionized calcium concentration varying, e.g., decreasing and/or increasing, faster than 0.05 mM/h.

In a 50th bis aspect according to any one of the previous aspects, the control unit (13) is configured to determine a variation of the measured concentration of the at least one substance in the second operating condition (ii) and to issue an alarm based on both (a) the measured value of concentration of the at least one substance in the second operating condition, and (b) the variation of the measured concentration of the at least one substance in the second operating condition.

In a 50th ter aspect according to the previous aspect, the control unit (13) is further configured to determine whether the measured value of concentration of the at least one substance in the second operating condition is closer to an upper threshold than to a lower threshold and whether the variation corresponds to an increase of the concentration and to issue the alarm in case an alarm criteria is matched.

For example, assuming that upper threshold is set to 1.20 mM a hypercalcemia risk exists if both (a) ionized calcium (iCa) is in the 'upper range', e.g., more than median value 1.05 mM, and (b) the increase rate is higher than e.g., 0.05 mM/h; this situation causes an alarm to be issued. Differently, no alarm is issued if ionized calcium iCa < 1.05 mM because a planned correction of too low ionized calcium (iCa) may be successfully implemented.

In a 50th quater aspect according to any one of the previous two aspects, the control unit (13) is further configured to determine whether the measured value of concentration of the at least one substance in the second operating condition is closer to a lower threshold than to an upper threshold and whether the variation corresponds to a decrease of the concentration and to issue the alarm in case an alarm criteria is matched.

For example, assuming that lower threshold is set to 0.9 mM a hypocalcemia risk exists if both (a) ionized calcium (iCa) is in the 'lower range', e.g., less than median value 1.05 mM, and (b) the decrease rate is higher than e.g., 0.05 mM/h; this situation causes an alarm to be issued. Differently, no alarm is issued if ionized calcium iCa > 1.05 mM because a planned correction of too high ionized calcium (iCa) may be successfully implemented.

In a 51st aspect according to any one of the previous aspects, the infusion solution (I) supplied by the infusion fluid source (10) includes at least one solute that reacts with a component present in the blood, such as citrate reacting with ionized calcium that is present in the blood, and wherein, in the first operating condition (i), the concentrate sensor (12) is configured to measure a reduction of the component that reacted with the solute in the infusion solution (I), in particular the concentration of ionized calcium after complexing with citrate.

In a 52nd aspect according to any one of the previous aspects, in the second operating condition (ii), the concentrate sensor (12) is configured to measure the patient systemic concentration of the at least one substance, in particular the patient systemic concentration of ionized calcium.

In a 53rd aspect according to any one of the previous aspects, the extracorporeal blood treatment apparatus (1) is a dialysis apparatus, such as a CRRT apparatus.

In a 54th aspect according to any one of the previous aspects, the extracorporeal blood treatment apparatus (1) includes a regional anticoagulation, in particular comprises a regional anticoagulation system, wherein the main infusion line (9) is part of the regional anticoagulation system.

In a 55th aspect according to any one of the previous aspects, the concentrate sensor (12) comprises a disposable portion connected to the blood circuit (6) and a fixed reusable portion connected to a body of the extracorporeal blood treatment apparatus (1) also supporting the main infusion pump (11), the disposable portion and the fixed reusable portion being removably connected when the concentrate sensor (12) is in use.

In a 56th aspect according to any one of the previous aspects, at least a disposable portion of the concentrate sensor (12) is part of a blood line accessory, the blood line accessory being either a separate part of the blood circuit (6) connectable to the blood withdrawal line (6a) or an integral element part of the blood withdrawal line (6a).

In a 57th aspect according to any one of the previous aspects, the control unit (13) is further configured to run a concentrate computation routine comprising the following steps:

- acquire the concentration of the at least one solute in the infusion fluid source (10);
- acquire an infusion flow rate through the main infusion line (9);
- acquire a blood flow rate through the blood circuit (6)
- activate the main infusion pump (11);
- acquire the signal from the concentrate sensor (12) when blood is infused with the infusion solution (I);
- calculate a patient systemic concentration of the at least one solute based on the signal from the concentrate sensor (12), the concentration of the at least one solute in the infusion fluid source (10), the infusion flow rate and the blood flow rate,
- wherein the patient systemic concentration of the at least one solute is the at least one solute concentration in the blood withdrawal line (6a) upstream the injection point (9a) of the main infusion line (9).

In a 58th aspect according to the previous aspect, said at least one solute is a substance not reacting, e.g., not complexing, with components in the infusion fluid source (10).

In a 59th aspect according to the previous two aspects, the concentrate computation routine is executed when the main infusion pump (11) is in the activated condition and the infusion solution (I) is injected into the blood withdrawal line (6a).

In a 60th aspect according to any one of the previous aspects, the extracorporeal blood treatment apparatus comprises: at least one auxiliary infusion line (14) connected to the blood withdrawal line (6a) of the extracorporeal blood circuit (6) between the injection point (9a) of the main infusion line (9) and the concentrate sensor (12); at least one auxiliary fluid source (15) of an auxiliary infusion solution (II) feeding fluid to the auxiliary infusion line (14); an auxiliary infusion pump (16) active on the auxiliary infusion line (14) and drivable between an activated condition wherein the auxiliary infusion pump (16) causes the auxiliary infusion solution (II) to flow from the corresponding auxiliary fluid source (15) to the blood withdrawal line (6a) of the extracorporeal blood circuit (6), and an inactivated condition, wherein the auxiliary infusion pump (16) is inactive and the auxiliary infusion solution (II) does not flow through the corresponding auxiliary infusion line (14); wherein the concentrate sensor (12) is placed on the blood withdrawal line (6a) between an auxiliary injection point (14a) of the auxiliary infusion line (14) and the filtration unit (2), the control unit (13) being operatively connected to the auxiliary infusion pump (16) of the auxiliary infusion line (14) to drive the auxiliary infusion pump (16) between the activated condition and the inactivated condition and respectively determine the concentrate sensor (12) to detect the concentration of at least one substance into blood flowing into the blood withdrawal line (6a) between the auxiliary injection point (14a) and the filtration unit (2).

In a 61st aspect according to any one of the previous aspects, the control unit (13) is configured to receive signals from the concentrate sensor (12) to determine the concentration of the at least one substance in first working condition (i), a second working condition, a third working condition and a fourth working condition of the extracorporeal blood treatment apparatus (1), wherein the control unit (13) is further configured to:

- in the first working condition (i):

  ○ activate the main infusion pump (11);
  ○ inactivate the auxiliary infusion pump (16);
  ○ acquire the signal from the concentrate sensor (12) to determine the concentration of the at least one substance into blood infused with the infusion solution (I);

- in the second working condition (ii):

  ○ inactivate the main infusion pump (11);
  ○ activate the auxiliary infusion pump (16);
  ○ acquire the signal from the concentrate sensor (12) to determine the concentration of the at least one substance into blood infused with the auxiliary infusion solution (II);

- in the third working condition (iii):

  ○ activate the main infusion pump (11);
  ○ activate the auxiliary infusion pump (16);
  ○ acquire the signal from the concentrate sensor (12) to determine the concentration of the at least one substance into blood infused with the infusion solution (II) and the auxiliary infusion solution (II);

- in the fourth working condition (iv):

  ○ inactivate the main infusion pump (11);
  ○ inactivate the auxiliary infusion pump (16);
  ○ acquire the signal from the concentrate sensor (12) to determine the concentration of the at least one substance into blood when no infusion solution (I, II) is infused into the blood.

In a 62nd aspect according to the previous aspect, the control unit (13) is configured to perform different sequences of at least two of the working conditions (i), (ii), (iii) and (iv) or different combination of different sequences of at least two of the working conditions (i), (ii), (iii) and (iv), optionally the control unit (13) being configured to repeat at least one sequence of the working conditions (i), (ii), (iii) and (iv), particularly a plurality of times during an extracorporeal blood treatment.

In a 63rd aspect according to any one of the previous aspects, in the first working condition (i), the control unit (13) is configured to keep the main infusion pump (11) in the activated condition and the auxiliary infusion pump (16) in the inactivated condition to allow the concentration sensor (12) to detect the at least one substance into blood infused with the infusion solution (I) supplied by the infusion fluid source (10) through the main infusion line (9).

In a 64th aspect according to the previous two aspects, in the first working condition (i), the control unit (13) is configured to keep the main infusion pump (11) in the activated condition and the auxiliary infusion pump (16) in the inactivated condition for a predetermined time period.

In a 65th aspect according to any one of the previous three aspects, in the first working condition (i), the control unit (13) is configured to acquire the signal from the concentrate sensor (12) to determine the concentration of at least one substance into blood infused with the infusion solution (I) after the main infusion pump (11) is in the activated condition and the auxiliary infusion pump (16) is in the inactivated condition for a time period which is at least a function of the blood flow rate on the blood withdrawal line (6a) of the extracorporeal blood circuit (6), in particular in correspondence of the concentrate sensor (12).

In a 66th aspect according to any one of the previous four aspects, in the first working condition (i), the control unit (13) is configured to acquire the signal from the concentrate sensor (12) to determine the concentration of at least one substance into blood infused with the infusion solution (I) after the main infusion pump (11) is in the activated condition and the auxiliary infusion pump (16) is in the inactivated condition for a time period which is at least a function of the cross section of the blood withdrawal line (6a) of the extracorporeal blood circuit (6), in particular the blood flow rate in correspondence of the concentrate sensor (12).

In a 67th aspect according to any one of the previous five aspects, in the first working condition (i), the control unit (13) is configured to acquire the signal from the concentrate sensor (12) to determine the concentration of at least one substance into blood infused with the infusion solution (I) after the main infusion pump (11) is in the activated condition and the auxiliary infusion pump (16) is in the inactivated condition for a time period which is at least a function of a distance on a portion of the blood withdrawal line (6a) of the extracorporeal blood circuit (6) between the injection point (9a, 14a) of the main infusion line (9) and/or the auxiliary infusion line (14) and the concentrate sensor (12).

In a 68th aspect according to any one of the previous six aspects, in the first working condition (i), the control unit (13) is configured to acquire the signal from the concentrate sensor (12) to determine the concentration of at least one substance into blood infused with the infusion solution (I) after the main infusion pump (11) is in the activated condition and the auxiliary infusion pump (16) is in the inactivated condition for a time period which is at least a function of the design of a portion of the withdrawal line (6a) of the extracorporeal blood circuit (6) between the injection point (9a, 14a) of the main infusion line (9) and/or the auxiliary infusion line (14) and the concentrate sensor (12).

In a 69th aspect according to any one of the previous seven aspects, in the first working condition (i), the control unit (13) is configured to acquire the signal from the concentrate sensor (12) to determine the concentration of at least one substance into blood infused with the infusion solution (I) after the main infusion pump (11) is in the activated condition and the auxiliary infusion pump (16) is in the inactivated condition for a time period which is at least a function of a volume of a portion of the blood withdrawal line (6a) of the extracorporeal blood circuit (6) between the injection point (9a, 14a) of the main infusion line (9) and/or the auxiliary infusion line (14) and the concentrate sensor (12).

In a 70th aspect according to any one of the previous eight aspects, in the first working condition (i), the control unit (13) is configured to acquire the signal from the concentrate sensor (12) to determine the concentration of at least one substance into blood infused with the infusion solution (I) after the main infusion pump (11) is in the activated condition and the auxiliary infusion pump (16) is in the inactivated condition for a time period which allows the concentrate sensor (12) to read a stable concentration value.

In a 71st aspect according to any one of the previous nine aspects, in the second working condition (ii), the control unit (13) is configured to keep the main infusion pump (11) in the inactivated condition and the auxiliary infusion pump (16) in the activated condition to allow the concentration sensor (12) to detect the at least one substance into blood infused with the auxiliary infusion solution (II) supplied by the auxiliary fluid source (16) through the auxiliary infusion line (14).

In a 72nd aspect according to the previous ten aspects, in the second working condition (ii), the control unit (13) is configured to keep the main infusion pump (11) in the inactivated condition and the auxiliary infusion pump (16) in the activated condition for a predetermined time period.

In a 73rd aspect according to any one of the previous eleven aspects, wherein, in the second working condition (ii), the control unit (13) is configured to acquire the signal from the concentrate sensor (12) to determine the concentration of at least one substance into blood infused with the auxiliary infusion solution (II) after the main infusion pump (11) is in the inactivated condition and the auxiliary infusion pump (16) is in the activated condition for a time period which is at least a function of the blood flow rate on the blood withdrawal line (6a) of the extracorporeal blood circuit (6), in particular in correspondence of the concentrate sensor (12).

In a 74th aspect according to any one of the previous twelve aspects, wherein, in the second working condition (ii), the control unit (13) is configured to acquire the signal from the concentrate sensor (12) to determine the concentration of at least one substance into blood infused with the auxiliary infusion solution (II) after the main infusion pump (11) is in the inactivated condition and the auxiliary infusion pump (16) is in the activated condition for a time period which is at least a function of the cross section of the blood withdrawal line (6a) of the extracorporeal blood circuit (6), in particular the blood flow rate in correspondence of the concentrate sensor (12).

In a 75th aspect according to any one of the previous thirteen aspects, in the second working condition (ii), the control unit (13) is configured to acquire the signal from the concentrate sensor (12) to determine the concentration of at least one substance into blood infused with the auxiliary infusion solution (II) after the main infusion pump (11) is in the inactivated condition and the auxiliary infusion pump (16) is in the activated condition for a time period which is at least a function of a distance on a portion of the blood withdrawal line (6a) of the extracorporeal blood circuit (6) between the injection point (14a) of the auxiliary infusion line (14) and the concentrate sensor (12).

In a 76th aspect according to any one of the previous fourteen aspects, in the second working condition (ii), the control unit (13) is configured to acquire the signal from the concentrate sensor (12) to determine the concentration of at least one substance into blood infused with the auxiliary infusion solution (II) after the main infusion pump (11) is in the inactivated condition and the auxiliary infusion pump (16) is in the activated condition for a time period which is at least a function of the design of a portion of the withdrawal line (6a) of the extracorporeal blood circuit (6) between the injection point (14a) of the auxiliary infusion line (14) and the concentrate sensor (12).

In a 77th aspect according to any one of the previous fifteen aspects, in the second working condition (ii), the control unit (13) is configured to acquire the signal from the concentrate sensor (12) to determine the concentration of at least one substance into blood infused with the auxiliary infusion solution (I) after the main infusion pump (11) is in the inactivated condition and the auxiliary infusion pump (16) is in the activated condition for a time period which is at least a function of a volume of a portion of the blood withdrawal line (6a) of the extracorporeal blood circuit (6) between the injection point (14a) of the auxiliary infusion line (14) and the concentrate sensor (12).

In a 78th aspect according to any one of the previous sixteen aspects, wherein in the second working condition (ii), the control unit (13) is configured to acquire the signal from the concentrate sensor (12) to determine the concentration of at least one substance into blood infused with the auxiliary infusion solution (II) after the main infusion pump (11) is in the inactivated condition and the auxiliary infusion pump (16) is in the activated condition for a time period which allows the concentrate sensor (12) to read a stable concentration value.

In a 79th aspect according to any one of the previous seventeen aspects, in the third working condition (iii), the control unit (13) is configured to keep the main infusion pump (11) in the activated condition and the auxiliary infusion pump (16) in the activated condition to allow the concentration sensor (12) to detect the at least one substance into blood infused with the infusion solution (I) supplied by the infusion fluid source (10) through the main infusion line (9) and the auxiliary infusion solution (II) supplied by the auxiliary fluid source (16) through the auxiliary infusion line (14).

In an 80th aspect according to the previous fourteen aspects, in the third working condition (iii), the control unit (13) is configured to keep the main infusion pump (11) in the activated condition and the auxiliary infusion pump (16) in the activated condition for a predetermined time period.

In an 81st aspect according to any one of the previous fifteen aspects, in the third working condition (iii), the control unit (13) is configured to acquire the signal from the concentrate sensor (12) to determine the concentration of at least one substance into blood infused with the infusion solution (I) and the auxiliary infusion solution (II) after the main infusion pump (11) is in the activated condition and the auxiliary infusion pump (16) is in the activated condition for a time period which is at least a function of the blood flow rate on the blood withdrawal line (6a) of the extracorporeal blood circuit (6), in particular in correspondence of the concentrate sensor (12).

In an 82nd aspect according to any one of the previous sixteen aspects, in the third working condition (iii), the control unit (13) is configured to acquire the signal from the concentrate sensor (12) to determine the concentration of at least one substance into blood infused with the infusion solution (I) and the auxiliary infusion solution (II) after the main infusion pump (11) is in the activated condition and the auxiliary infusion pump (16) is in the activated condition for a time period which is at least a function of the cross section of the blood withdrawal line (6a) of the extracorporeal blood circuit (6), in particular the blood flow rate in correspondence of the concentrate sensor (12).

In an 83rd aspect according to any one of the previous seventeen aspects, in the third working condition (iii), the control unit (13) is configured to acquire the signal from the concentrate sensor (12) to determine the concentration of at least one substance into blood infused with the infusion solution (I) and the auxiliary infusion solution (II) after the main infusion pump (11) is in the activated condition and the auxiliary infusion pump (16) is in the activated condition for a time period which is at least a function of a distance on a portion of the blood withdrawal line (6a) of the extracorporeal blood circuit (6) between the injection point (9a, 14a) of the main infusion line (9) and/or the auxiliary infusion line (14) and the concentrate sensor (12).

In an 84th aspect according to any one of the previous eighteen aspects, in the third working condition (iii), the control unit (13) is configured to acquire the signal from the concentrate sensor (12) to determine the concentration of at least one substance into blood infused with the infusion solution (I) and the auxiliary infusion solution (II) after the main infusion pump (11) is in the activated condition and the auxiliary infusion pump (16) is in the activated condition for a time period which is at least a function of the design of a portion of the blood withdrawal line (6a) of the extracorporeal blood circuit (6) between the injection point (9a, 14a) of the main infusion line (9) and/or the auxiliary infusion line (14) and the concentrate sensor (12).

In an 85th aspect according to any one of the previous nineteen aspects, in the third working condition (iii), the control unit (13) is configured to acquire the signal from the concentrate sensor (12) to determine the concentration of at least one substance into blood infused with the infusion solution (I) and the auxiliary infusion solution (I) after the main infusion pump (11) is in the activated condition and the auxiliary infusion pump (16) is in the activated condition for a time period which is at least a function of a volume of a portion of the blood withdrawal line (6a) of the extracorporeal blood circuit (6) between the injection point (9a, 14a) of the main infusion line (9) and/or the auxiliary infusion line (14) and the concentrate sensor (12).

In an 86th aspect according to any one of the previous twenty aspects, in the third working condition (iii), the control unit (13) is configured to acquire the signal from the concentrate sensor (12) to determine the concentration of at least one substance into blood infused with the infusion line (I) and the auxiliary infusion solution (II) after the main infusion pump (11) is in the activated condition and the auxiliary infusion pump (16) is in the activated condition for a time period which allows the concentrate sensor (12) to read a stable concentration value.

In an 87th aspect according to any one of the previous twenty-one aspects, in the fourth working condition (iv), the control unit (13) is configured to keep the main infusion pump (11) and the auxiliary infusion pump (16) in the inactivated condition to allow blood with no infusion solution (I, II) infused by the main infusion line (9) and/or by auxiliary infusion line (14) to reach the concentration sensor (12), the control unit (13) detecting the concentration of the at least one substance into blood when blood with no infusion solution (I, II) reaches the concentration sensor (12).

In an 88th aspect according to any one of the previous twenty-two aspects, in the fourth working condition (iv), the control unit (13) is configured to keep the main infusion pump (11) and the auxiliary infusion pump (16) in the inactivated condition for a predetermined time period.

In an 89th aspect according to any one of the previous twenty-three aspects, in the fourth working condition (iv), the control unit (13) is configured to keep the main infusion pump (11) and the auxiliary infusion pump (16) in the inactivated condition for a time period which is at least a function of the blood flow rate on the withdrawal line (6a) of the extracorporeal blood circuit (6), in particular in correspondence of the concentrate sensor (12).

In a 90th aspect according to any one of the previous twenty-four aspects, in the fourth working condition (iv), the control unit (13) is configured to keep the main infusion pump (11) and the auxiliary infusion pump (16) in the inactivated condition for a time period which is at least a function of the cross section of the withdrawal line (6a) of the extracorporeal blood circuit (6), in particular in correspondence of the concentrate sensor (12).

In a 91st aspect according to any one of the previous twenty-five aspects, wherein, in the fourth working condition (iv), the control unit (13) is configured to keep the main infusion pump (11) and the auxiliary infusion pump (16) in the inactivated condition for a time period which is at least a function of a distance on the withdrawal line (6a) of the extracorporeal blood circuit (6) between the injection point (9a, 14a) of the main infusion line (9) and/or the auxiliary infusion line (14) and the concentrate sensor (12).

In a 92nd aspect according to any one of the previous twenty-six aspects, wherein, in the fourth working condition (iv), the control unit (13) is configured to keep the main infusion pump (11) and the auxiliary infusion pump (16) in the inactivated condition for a time period which is at least a function of the design of a portion of the withdrawal line (6a) of the extracorporeal blood circuit (6) between the injection point (9a, 14a) of the main infusion line (9) and/or the auxiliary infusion line (14) and the concentrate sensor (12).

In a 93rd aspect according to any one of the previous twenty-seven aspects, in the fourth working condition (iv), the control unit (13) is configured to keep the main infusion pump (11) and the auxiliary infusion pump (16) in the inactivated condition for a time period which is at least a function of a volume of a portion of the withdrawal line (6a) of the extracorporeal blood circuit (6) between the injection point (9a, 14a) of the main infusion line (9) and/or the auxiliary infusion line (14) and the concentrate sensor (12).

In a 94th aspect according to any one of the previous twenty-eight aspects, in the fourth working condition (iv), the control unit (13) is configured to keep the main infusion pump (11) and the auxiliary infusion pump (16) in the inactivated condition for a time period which allows the concentrate sensor (12) to read a stable concentration value.

In a 95th according to any one of the previous twenty-nine aspects, wherein the at least one substance the concentrate sensor (12) is configured to detect into blood or blood infused with the infusion solution (I) supplied by the infusion fluid source (10) or blood infused with the auxiliary infusion solution (II) supplied by the auxiliary fluid source (15) or into blood infused with the infusion solution (I) supplied by the infusion fluid source (10) and the auxiliary infusion solution (II) supplied by the auxiliary fluid source (15) is at least ionized calcium.

In a 96th aspect according to any one of the previous forty aspects, wherein the concentrate sensor (12) is configured to detect the concentration of two different substances into blood or blood infused with the infusion solution (I) supplied by the infusion fluid source (10) or blood infused with the auxiliary infusion solution (II) supplied by the auxiliary fluid source (15) or into blood infused with the infusion solution (I) supplied by the infusion fluid source (10) and the auxiliary infusion solution (II) supplied by the auxiliary fluid source (15), in particular one of the two different substances being ionized calcium.

In a 97th aspect according to any one of the previous forty-one aspects, the concentrate sensor (12) is configured to separately detect the concentration of two different substances into blood or blood infused with the infusion solution (I) supplied by the infusion fluid source (10) or blood infused with the auxiliary infusion solution (II) supplied by the auxiliary fluid source (15) or into blood infused with the infusion solution (I) supplied by the infusion fluid source (10) and the auxiliary infusion solution (II) supplied by the auxiliary fluid source (15), in particular one of the two different substances being ionized calcium.

In a 98th aspect according to any one of the previous aspects from 59th to 93th, herein the concentrate sensor (12) is configured to simultaneously detect the concentration of two different substances into blood or blood infused with the infusion solution (I) supplied by the infusion fluid source (10) or blood infused with the auxiliary infusion solution (II) supplied by the auxiliary fluid source (15) or into blood infused with the infusion solution (I) supplied by the infusion fluid source (10) and the auxiliary infusion solution (II) supplied by the auxiliary fluid source (15), in particular one of the two different substances being ionized calcium.

In a 99th aspect according to any of the previous forty-three aspects, wherein the infusion solution (I) supplied by the infusion fluid source (10) includes at least an anticoagulant citrate.

In a 100th aspect according to any one of the previous forty-four aspects, wherein the control unit (13) is configured to adjust: the amount of the auxiliary infusion solution (II) supplied by the auxiliary fluid source (15) to be infused into the withdrawal line (6a) on the base of the concentration of at least one substance detected by the concentrate sensor (12); or, the amount of the infusion solution (I) supplied by the infusion fluid source (10) to be infused into the withdrawal line (6a) and the amount of the auxiliary infusion solution (II) supplied by the auxiliary fluid source (15) to be infused into the withdrawal line (6a) on the base of the concentration of at least one substance detected by the concentrate sensor (12).

In a 101st aspect according to any one of the previous forty-six aspects, the auxiliary fluid source (14) comprises at least one bag.

In a 102nd aspect according to any one of the previous forty-seven aspects, the auxiliary fluid source (14) comprises at least one bag made of a plastic material.

In a 103rd aspect according to any one of the previous forty-eight aspects, the bag of the auxiliary fluid source (14) is flexible and made of two opposite leaves joined each other, optionally hermetically sealed, in particular welded each other.

In a 104th aspect according to any one of the previous forty-nine aspects, the bag of the auxiliary fluid source (10) is disposable.

In a 105th aspect according to any one of the previous fifty aspects, comprising a blood pump (17), wherein the blood pump (17) is placed between an injection point (14a) of the auxiliary infusion line (14) and the filtration unit (2).

In a 106th aspect according to the previous fifty-one aspects, wherein the blood pump (17) is placed upstream the concentrate sensor (12).

In a 107th aspect according to any one of the previous fifty-two aspects, the auxiliary infusion solution (II) comprises a substitute solution, optionally including at least one buffer agent.

In a 108th aspect according to any one of the previous aspects, the at least one substance the concentrate sensor (12) is configured to detect into blood or blood infused with the infusion solution (I) supplied by the infusion fluid source (10) is a blood hematocrit, hemoglobin, urea and/or creatinine. The possibility to measure hematocrit, hemoglobin, urea and/or creatinine in its systemic concentration (i.e., when the infusion of fluid is stopped) allows both to check the patient actual systemic value during the treatment and also to check whether any 'calculated' or 'estimated' systemic value of the concentration of the same blood parameter (by a mathematical model for example) is correct or should be corrected.

In a 109th aspect according to any one of the aspects 2nd to 108th when they depend on the 2nd aspect, said at least one solute is a substance not reacting, e.g., not complexing, with components in the infusion fluid source (10).

In a 110th aspect according to the any of the aspects 2nd to 106th when they depend on the 2nd aspect, wherein the concentrate computation routine is executed when the main infusion pump (11) is in the activated condition and the infusion solution (I) is injected into the blood withdrawal line (6a).

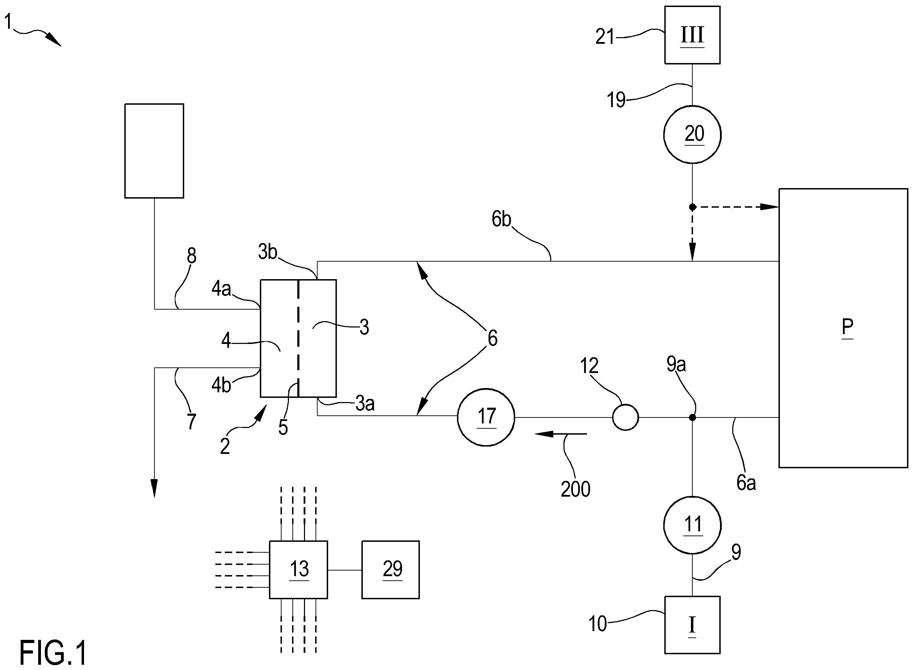

Additionally, any of the above aspects, or portions thereof, and/or any of the features, functionality and alternatives described in connection with any one or more of Figs. 1 to 6 may be combined with any of the features, functionality and alternatives described in connection with any other of Figs. 1 to 6.

It is accordingly an advantage of the present disclosure to provide an extracorporeal blood treatment apparatus configured for obtaining concentration measures on the blood lines with a straightforward design resulting less complex and less expensive than the traditional prior art extracorporeal blood treatment apparatuses.

It is another advantage of the present disclosure to provide an extracorporeal blood treatment apparatus easier to be monitored than the traditional prior art extracorporeal blood treatment apparatuses

It is yet a further advantage of the present disclosure to provide an extracorporeal blood treatment apparatus, wherein one or more solute concentration in the blood is monitored a plurality of times during a treatment allowing a better treatment accuracy than the one achieved by the traditional prior art extracorporeal blood treatment apparatuses.

Additional features and advantages are described in, and will be apparent from, the following Detailed Description and the Figures. The features and advantages described herein are not allinclusive and, in particular, many additional features and advantages will be apparent to one of ordinary skill in the art in view of the figures and description. Also, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the inventive subject matter.

BRIEF DESCRIPTION OF THE FIGURES

Figure 1 is a schematic view of a fluid circuit of an extracorporeal blood treatment apparatus according to a first embodiment of the present invention;
Figure 2 is a schematic view of a fluid circuit of an extracorporeal blood treatment apparatus according to a second embodiment of the present invention;
Figure 3 is a schematic view of a fluid circuit of an extracorporeal blood treatment apparatus according to a third embodiment of the present invention;
Figure 4 is another schematic view of a fluid circuit of an extracorporeal blood treatment apparatus according to a fourth embodiment of the present invention;
Figure 5 is a first block diagram representing a monitoring method for monitoring at least one substance into a fluid, in particular blood, according to the present application;
Figure 6 is a second block diagram representing a variant of the monitoring method for monitoring at least one substance into a fluid, in particular blood, according to the present application.

DETAILED DESCRIPTION

Extracorporeal blood treatment (for example, but not exclusively, renal failure dialysis) may be used in patients with rapidly developing loss of kidney function, called acute renal failure or slowly worsening kidney function, called Stage 5 chronic kidney disease (or end-stage renal disease). In the following description, some embodiments of extracorporeal blood treatment apparatuses will be firstly described being suitable, or designed, principally (though not exclusively) for intensive care treatments.

Unless defined otherwise, all technical and scientific terms used have the same meaning as commonly understood by one of ordinary skill in the art.

With reference to the attached figures, the numeral 1 globally refers to an extracorporeal blood treatment apparatus which may be used for performing any kind of kidney failure therapy, such as Hemodialysis ("HD"), Hemofiltration ("HF"), Hemodiafiltration ("HDF"), Hemoperfusion and in the context of continuous renal replacement therapies (CRRT) with or without anticoagulation, for example CRRT with or without systemic anticoagulation (e.g., heparin) / with or without regional anticoagulation (e.g., citrate).

With reference to figure 1, the extracorporeal blood treatment apparatus 1 comprises a filtration unit 2, of the type having a primary chamber 3 and a secondary chamber 4 separated by a semi-permeable membrane 5, an extracorporeal blood circuit 6 comprising a blood withdrawal line 6a connected to an inlet 3a of the primary chamber 3 of the filtration unit 2, and a blood return line 6b connected to an outlet 3b of the primary chamber 3 of the filtration unit 2.

Optionally, the extracorporeal blood treatment apparatus 1 includes a dialysate line 7 connected to an outlet 4b of the secondary chamber 4 and a supply line 8 connected to an inlet 4a of the secondary chamber 4.

The extracorporeal blood treatment apparatus 1 further comprises at least one main infusion line 9 connected, on one side, to the blood withdrawal line 6a of the extracorporeal blood circuit 6, at an injection point 9a, to transfer an infusion solution I into blood, and, on the other side, to at least one infusion fluid source 10 of the infusion solution I. A main infusion pump 11 is active on the main infusion line 9 and drivable between an activated condition wherein the main infusion pump 11 causes the infusion solution I to flow from the corresponding infusion fluid source 10 to the blood withdrawal line 6a of the extracorporeal blood circuit 6, and an inactivated condition, wherein the main infusion pump 11 is inactive and the infusion solution I does not flow through the corresponding main infusion line 9. The infusion pump 11 may be an occlusive pump (such as a peristaltic pump) that does not allow fluid to flow inside the line when it is stopped. Alternatively, or in combination, a clamp (or any other fluid blocking element) may be placed on the infusion line to guarantee that no fluid flows inside the infusion line when in the inactivated condition.

The extracorporeal blood treatment apparatus 1 also comprises a concentrate sensor 12 placed on the blood withdrawal line 6a of the extracorporeal blood circuit 6 between the injection point 9a of the main infusion line 9 and the filtration unit 2 for monitoring the concentration of at least one substance S detectable into blood flowing into the blood withdrawal line 6a between the injection point 9a and the filtration unit 2.

The concentrate sensor 12 may be any sensor suitable to detect the concentration of the at least one substance. For example, the sensor comprises an optical concentrate sensor, optionally a fluorescence optical concentrate sensor; the concentrate sensor may also require micro sampling.

In order to perform the monitoring operations through the concentrate sensor 12, the extracorporeal blood treatment apparatus 1 is provided with a control unit 13 operatively connected to the concentration sensor 12 and the main infusion pump 11 of the main infusion line 9, through known means. The control unit 13 is configured to drive the main infusion pump 11 between the activated condition and the inactivated condition and to receive signals from the concentrate sensor 12 to determine the concentration of the at least one substance S in a first (i) and in a second (ii) operating condition of the extracorporeal blood treatment apparatus 1.

In particular, the control unit 13 is configured to perform the following operations: (i) in the first operating condition, the control unit 13 activates the main infusion pump 11 and acquires at least one signal from the concentrate sensor 12 to determine the concentration of the at least one substance S into blood infused with the infusion solution I (i.e., to determine the concentration of mixed blood combination of the infused solution and of the substance S coming from the patient); and, (ii) in the second operating condition, the control unit 13 inactivates the main infusion pump 11 and acquires at least one signal from the concentrate sensor 12 to determine the concentration of the at least one substance S, in particular the same substance S detected during the first operating condition (i), into blood when no infusion solution I is infused into the blood.

The first (i) and the second operating condition (ii) may be repeated a plurality of times during an extracorporeal blood treatment, in particular according to a sufficient number of times to allow a proper monitoring of the patient and/or the operation of the blood treatment apparatus and eventually also to establish the trend over time of the measured data.

It has to be noted that, in the second operating condition (ii), the control unit 13 is configured to keep the main infusion pump 11 in the inactivated condition to allow blood with no infusion solution I infused by the main infusion line 9 to reach the concentration sensor 12. In this way, the control unit 13 detects the concentration of the substance to be monitored into blood when blood with no infusion solution I reaches the concentration sensor 12. In other words, in the second operating condition (ii) the control unit 13 acquires the concentration measurements, provided by the concentration sensor 12 of the corresponding substance to be detected included into the blood coming from the patient P systemic concentration and not varied by any infusion.

The control unit 13 may be configured to keep the main infusion pump 11 in the inactivated condition for a predetermined time period to allow the blood without the infusion solution to reach the sensor; the predetermined time period may be fixed or variable.

The control unit 13 may be configured to keep the main infusion pump 11 in the inactivated condition for a time period which is at least a function of:

- the blood flow rate in the blood withdrawal line 6a of the extracorporeal blood circuit 6, in particular a blood flow rate in correspondence of the concentrate sensor 12; and/or,

- a volume of a portion of the withdrawal line 6a of the extracorporeal blood circuit between the injection point 9a of the main infusion line 9 and the concentrate sensor 12.

Alternatively or in combination, in the second operating condition (ii), the control unit 13 is configured to keep the main infusion pump 11 in the inactivated condition for a time period which allows the concentrate sensor 12 to read a stable concentration value.

In the first operating condition (i), the control unit 13 is configured to keep the main infusion pump 11 in the activated condition to allow the concentration sensor 12 to detect the at least one substance S into blood infused with the infusion solution I supplied by the infusion fluid source 10 through the main infusion line 9.

In this situation, i.e., in the first operating condition (i), the control unit 13 is configured to keep the main infusion pump 11 in the activated condition for substantially the entire treatment except when concentration of the substance to be monitored into blood not infused with infusion solution I is requested.

The control unit 13 may also be configured to acquire the signals from the concentrate sensor 12 to determine the concentration of the at least one substance into blood infused with the infusion solution I after the main infusion pump 11 is in the activated condition for a time period:

- which is at least a function of the blood flow rate on the blood withdrawal line 6a of the extracorporeal blood circuit 6, in particular the blood flow rate in correspondence of the concentrate sensor 12; and/or,

- which is at least a function of a volume of a portion of withdrawal line 6a of the extracorporeal blood circuit 6 between the concentrate sensor 12 and the injection point 9a of the main infusion line 9; and/or;

- which allows the concentrate sensor 12 to read a stable concentration value.

The concentrate sensor 12 may be configured to detect different kinds of substances S inside blood not infused with any infusion solution I or blood infused with the infusion solution I supplied by the infusion fluid source 10. Among the substances S the concentrate sensor 12 may be configured to detect, a blood electrolyte such as sodium, potassium, phosphates, magnesium, chloride is included. In particular, the concentrate sensor 12 is configured to detect at least ionized calcium.

It is also possible to provide a concentrate sensor 12 configured to detect the concentration of two or more different substances S into blood not infused with any infusion solution I or blood infused with the infusion solution I supplied by the infusion fluid source 10, wherein one of the two different substances S is ionized calcium. In this particular case, the concentrate sensor 12 may be configured to separately detect the concentration of two different substances S in sequence both into blood not infused with any infusion solution I and into blood infused with the infusion solution I supplied by the infusion fluid source 10. Alternatively, the concentrate sensor 12 may be configured to simultaneously detect the concentration of two different substances S into blood not infused with any infusion solution I and into blood infused with the infusion solution I supplied by the infusion fluid source 10.

Though not limitative, the extracorporeal blood treatment apparatus 1 comprises only one concentrate sensor 12 on the extracorporeal blood circuit 6 which is configured to detect at least ionized calcium into blood not infused with any infusion solution I or blood infused with the infusion solution I supplied by the infusion fluid source 10 and it does not comprise other ion concentrate sensors on the blood circuit itself.

The control unit 13 may be configured to adjust the amount of the infusion solution I supplied by the infusion fluid source 10 to be infused into the withdrawal line 6a on the base of the concentration of the corresponding substance S to be detected by the concentrate sensor 12, in particular based on the concentration measured during the first operating condition (i).

It has to be noted that the infusion solution I supplied by the infusion fluid source 10 may include at least one solute that reacts with a component present in the blood, such as citrate reacting with calcium (and with magnesium, too) that is present in the blood.

In the first operating condition (i), the concentrate sensor 12 is configured to measure a reduction of the component that reacted with the solute in the infusion solution I, in particular the concentration reduction of ionized calcium after a part of calcium ions had complexed with citrate.

In the second operating condition (ii), the concentrate sensor 12 is configured to measure the patient systemic concentration of the at least one substance, in particular the patient systemic concentration of ionized calcium.

The infusion fluid source 10 comprises at least one (in particular disposable) bag, optionally made of a plastic material, particularly flexible and made of two opposite leaves joined (hermetically sealed), each other, in particular welded each other.

Looking at figure 1, it is possible to see that the extracorporeal blood treatment apparatus 1 comprises a blood pump 17 active on the extracorporeal blood circuit 6. The blood pump 17 is placed on the withdrawal line 6a of the extracorporeal blood circuit 6, in particular between the injection point 9a of the main infusion line 9 and the filtration unit 2, more in particular between the concentrate sensor 12 and the filtration unit 2. The blood flow is indicated in figure 1 with arrow 200.

The extracorporeal blood treatment apparatus 1 further comprises an ion replenishment infusion line 19 provided with an ion replenishment infusion pump 20 and a fluid source 21 of ion replenishment solution III connected to one end of the ion replenishment infusion line 19. The ion replenishment infusion line 19 is able to infuse the ion replenishment solution III either into the blood return line 6b or directly into the patient P. Optionally, the ion replenishment solution III may comprise ionized calcium, specifically a concentrated solution of ionized calcium, that is infused in order to reestablish physiological concentration of ionized calcium in the patient blood. The ion replenishment solution III may further comprise magnesium, specifically a concentrated solution of magnesium.

The control unit 13 may be configured to adjust the amount of the ion replenishment solution III supplied by the corresponding fluid source 16 of ion replenishment solution III on the base of the concentration of a corresponding substance detected by the concentrate sensor 12, in particular based on the concentration of such substance measured during the second operating condition (ii), such as the patient systemic concentration of ionized calcium measured during the second operating condition (ii).

The extracorporeal blood treatment apparatus 1 comprises a user interface comprising a screen for displaying data pertaining to the extracorporeal blood treatment apparatus 1. The measured concentrations of one or more substances in the first (i) and in the second (ii) operating condition of the extracorporeal blood treatment apparatus 1 are displayed on the screen, in particular versus one or more different substances monitored and/or being monitored.

Optionally the measured concentrations of one or more substances to be detected in the first operating condition (i) and/or the measured concentrations of one or more substances in the second operating condition (ii) are displayed as a chart over time.

The control unit 13 may also be configured to display the measured concentrations of the substance (particularly measured during the first operating condition), for example the ionized calcium, as a function of the anticoagulant dose, for example the citrate dose. Furthermore, systemic ionized calcium iCa may be displayed in combination with solution III infusion data (especially as a function of a calcium compensation index, such as compensation percentage, CaComp in the context of regional anticoagulation settings.

The control unit 13 is configured to compare the measured concentration of the at least one substance to be detected in the first operating condition (i) with a first upper threshold and to issue an alarm in case the measured concentration exceeds the first upper threshold, in particular when the ionized calcium concentration is over the first upper threshold, i.e., higher than 0.40 mM. Indeed, in case the ionized calcium is above 0.40 mM there might be a risk that the coagulation cascade may work and that part of the blood coagulate inside the extracorporeal blood circuit.

At the same time a too low value of ionized calcium (e.g., below 0,25 Mm) indicates that the anticoagulant infusion may be reduced. Therefore, in the first operating condition (i). the control unit 13 may be also configured to compare the measured concentration of the substance with a first low threshold and to issue an alarm in case the measured concentration exceeds the first low threshold, in particular the alarm being issued if ionized calcium concentration is below the first low threshold, such as ionized calcium lower than 0.25 mM.

Similarly, the control unit 13 is configured to compare the measured concentration of the same substance in the second operating condition (ii) with a second lower threshold and to issue an alarm in case the measured concentration exceeds the second lower threshold, in particular when the ionized calcium concentration is below the second lower threshold, i.e., lower than 0.90 mM. Ionized calcium below 0.90 mM inside the patient would not be a physiological value and the disequilibrium may affect the patient health.

Moreover, the control unit 13 is configured to compare the measured concentration of the substance in the second operating condition (ii) with a second upper threshold and to issue an alarm in case the measured concentration exceeds the second upper threshold, in particular the alarm being issued if ionized calcium concentration is beyond the second upper threshold, such as ionized calcium higher than 1.20 mM.

The control unit 13 is configured to: determine a variation of the measured concentration of at least one substance in the second operating condition (ii); compare the determined variation with a drift threshold; and, issue an alarm in case the variation exceeds the drift threshold, when the detected ionized calcium concentration is varying faster than the drift threshold, e.g. decreasing and/or increasing faster than 0.05 mM/h. Indeed, a fast variation rate of ionized calcium may affect the delicate electrolyte equilibrium of the patient and also bring quickly the solute concentration to dangerous values.

Additionally, the control unit 13 may be further configured to determine whether, in the second operating condition, the measured value of concentration of the substance is closer to an upper threshold than to a lower threshold and whether the variation corresponds to an increase of the concentration and to issue the alarm in case an alarm criteria is matched.

For example, assuming that upper threshold is set to 1.20 mM (and lower threshold at 0.9) a hypercalcemia risk exists if both (a) ionized calcium (iCa) is in the 'upper range', e.g., more than median value 1.05 mM, and (b) the increase rate is higher than e.g., 0.05 mM/h; this situation causes an alarm to be issued. Differently, no alarm is issued if ionized calcium iCa < 1.05 mM because a planned correction of too low ionized calcium (iCa) may be successfully implemented.

Correspondingly, in the second operating condition, the control unit 13 is further configured to determine whether the measured value of concentration of the substance is closer to a lower threshold than to an upper threshold and whether the variation corresponds to a decrease of the concentration and to issue the alarm in case an alarm criteria is matched.

For example, assuming that lower threshold is set to 0.9 mM a hypocalcemia risk exists if both (a) ionized calcium (iCa) is in the 'lower range', e.g., less than median value 1.05 mM, and (b) the decrease rate is higher than e.g., 0.05 mM/h; this situation causes an alarm to be issued. Differently, no alarm is issued if ionized calcium iCa > 1.05 mM because a planned correction of too high ionized calcium (iCa) may be successfully implemented.

Alternatively to what has been disclosed above, the control unit 13 may be configured to drive the main infusion pump 11 between the activated condition and the inactivated condition and to receive signals from the concentrate sensor 12 to determine the concentration of the at least one solute.

In another embodiment that may be also used in combination with the measuring procedure described above, the control unit 13 is further configured determine the concentration of a substance in the blood that does not react with the infusion solution, but whose concentration is in any case affected by the infusion solution. Indeed, a variation of the concentration of a substance in the extracorporeal blood may occur due to the substance reacting with another substance present in the infusion solution (e.g., ionized calcium in the blood complexing with citrate in the infusion solution) or because additional substance is added with the infusion solution (e.g., sodium concentration in the blood changing - increasing or decreasing - due to sodium concentration of fluid injected through the infusion line). Of course both effects may be present at the same time.

In this additional embodiment, the control unit 13 is further configured to run a concentrate computation routine comprising the following steps:

- acquire the concentration of the at least one solute in the infusion fluid source 10;
- acquire an infusion flow rate through the main infusion line 9;

- acquire a blood flow rate through the blood circuit 6;
- activate the main infusion pump 11;
- acquire the signal from the concentrate sensor 12 when blood is infused with the infusion solution I;
- calculate a patient systemic concentration of the at least one solute based on the signal from the concentrate sensor 12, the concentration of the at least one solute in the infusion fluid source 10, the infusion flow rate and the blood flow rate.

It is noted that, depending on the solute characteristics, namely whether it distributes in whole blood (including within red blood cells) or in plasma only, the computation process may have to further consider blood hematocrit as a relevant parameter to take into account.

The patient systemic concentration of the corresponding solute is the corresponding solute concentration in the blood withdrawal line 6a upstream the injection point 9a of the main infusion line 9.

In this case, the solute is a substance not reacting. For instance, the solute is a substance not complexing with components in the infusion fluid source 10.

Therefore, the computation routine is executed when the main infusion pump 11 is in the activated condition and the infusion solution (I) is injected into the blood withdrawal line 6a of the extracorporeal blood circuit 6. Indeed, in this latter case, the patient systemic concentration of the substance may be calculated starting from the sensor measurement and subtracting the effects of the infusion fluid injected into the extracorporeal blood circuit.

According to a further aspect of the present application the main infusion line 9 may be a part of a regional anticoagulation system.

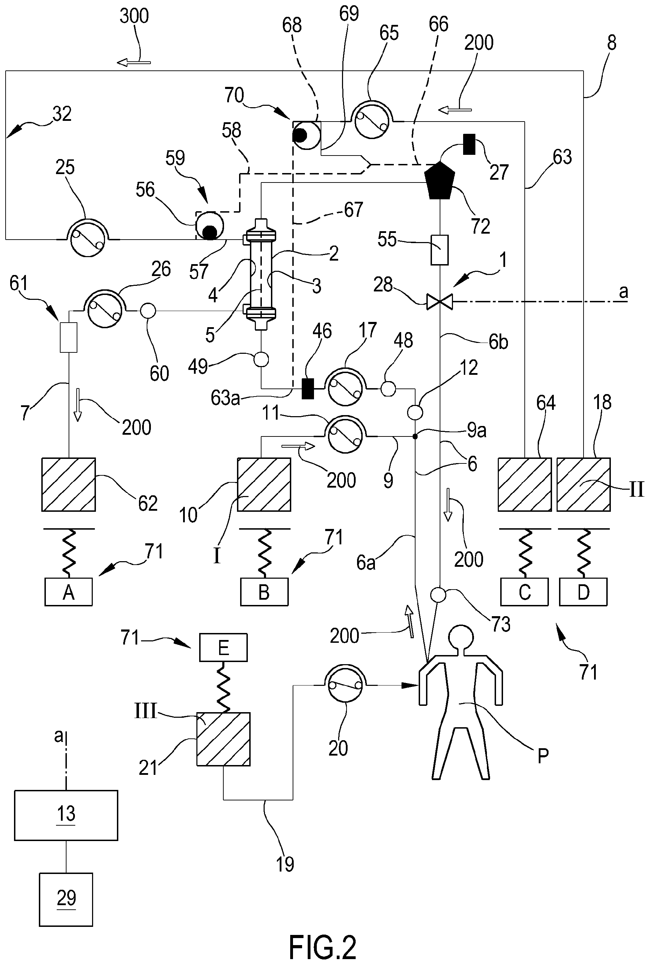

With reference to the embodiment schematically shown in figure 2, it is shown another example of an extracorporeal blood treatment apparatus 1 particularly, but not exclusively, designed for CRRT treatments.

With reference to figure 2, the numeral 1 globally refers to the extracorporeal blood treatment apparatus, in particular for intensive care therapies. The apparatus according to figure 2 is particularly designed for continuous renal replacement therapies (CRRT). CRRT systems are configured for delivering very specific treatments designed for patients versing in acute states of illness and who have temporarily lost their kidney function in its entirety. In this respect, CRRT systems may be structurally and/or operationally different from extracorporeal blood treatment systems designed for chronic patient care. In contrast to chronic patients, acute patients temporarily experience complete loss of their kidney function typically due to a contemporaneous state of severe injury or during recovery from surgery. Consequently, acute patients are often extremely weak and typically not in a condition to be submitted to regular dialysis treatment, which could further deteriorate their state and lead to serious and possibly life-threatening complications.

Under circumstances as described, CRRT systems are designed to individually treat a patient exhibiting very poor health, without inducing further stress to the patient body, in particular without allowing vital parameters pertaining to the patient's blood to deviate from ideal or near-ideal values. Within the scope of this document CRRT systems are, thus, inherently characterized by one or more of the following features. CRRT involves renal replacement therapy, meaning an adjuvant therapy aimed firstly at facilitating continuous fluid removal in diuretic-resistant or acute renal failure patients. Therefore, CRRT systems inherently require a continuous net fluid removal from the patient. In other words, a CRRT system requires a fluid balance control system, such as a weight loss control system, configured to generate a continuous net weight loss rate (as opposed to merely controlling parameters to enable achieving a desired target weight loss as typically found in chronic patient care). Furthermore, acute patients experience extravascular fluid overload, which cannot be safely removed within a short period of time (e.g. within a few hours of chronic treatment) without causing potentially severe consequences (e.g. hypovolemic shock, arrhythmia, hypoxemia, hypoventilation, etc.). Therefore, a CRRT system must inherently include a much more accurate control over system parameters, in particular flow rates, in order to ensure that the required low flow rates of both blood circulating extracorporeally and of treatment fluid (infused in the extracorporeal circuit or diffused through the dialyzer) are used. Moreover, CRRT treatment is performed continuously (e.g. for days or even weeks, without interruption/with minimal interruptions). Therefore, treatment settings in CRRT are based on flow rate settings, rather than settings pertaining to some specified treatment time (which would be unknown as acute patients may require treatment for an unknown time). Consequently, operation of CRRT systems cannot be based on some pre-defined absolute weight loss to be achieved, but rather on a meticulously controlled fluid balance in the patient, requiring continuous adjustments to a number of operating parameters, which have to be controlled and maintained during the entire (and a priori unknown) treatment time, based on a set weight-loss rate. Additionally, CRRT renal replacement therapy involves therapy substituting kidney functions for a relatively long time period and, thus, a CRRT system further requires at least either fresh dialysis liquid exchange in the dialyzer (in order to remove unwanted substances from blood and to add desired substances to the blood by diffusion) and/or fresh infusion fluid in combination with ultrafiltration (in order to remove unwanted substances from blood and to add desired substances to the blood by convection). At least for the reasons set forth above, CRRT systems need to exhibit specific technical features enabling the system to:

- allow setting of a weight loss rate;
- continuously remove excess water in accordance with a set weight loss rate;

- operate continuously at comparably low flow rates compatible with CRRT, and
- Balance ion equilibrium by means of proper dialysis being performed and/or by means of substitution fluid continuously being delivered at controlled flow rates.

The apparatus 1 of figure 2 has an extracorporeal blood circuit 6, which takes blood from a patient P, for instance through a catheter introduced into a vein or artery of said patient, and through the blood withdrawal line 6a takes said blood, for instance continuously, to the filtration unit 2. The blood passes through the primary chamber 3 of the filtration unit 2 and, through the blood return line 6b, the treated blood is carried back to the patient P.

In the example of figure 2, the connection with a main infusion line 9, in particular an anticoagulant line, is provided immediately downstream from the blood collecting zone on the blood withdrawal line 6a. In particular, the apparatus 1 is equipped with an infusion fluid source 10 of an infusion solution I, which in particular corresponds to a regional anticoagulant, such as at least a fluid container or bag for supplying the main infusion line 9, by using corresponding means for conveying fluid, in the example shown comprising an infusion pump 11, for instance a peristaltic pump.

It is possible to control the fluid flow within said line by introducing the infusion solution (regional anticoagulant) directly into the blood through a direct connection to the blood withdrawal line 6a. After defining a direction of fluid (blood) circulation 200 (during normal use of the apparatus 1) from the blood withdrawal line 6a towards the filtration unit 2 and from the latter through the blood return line 6b towards the patient P, a known blood pressure sensor 48, which shall not be described in further detail, is placed downstream the main infusion line 9.

The extracorporeal blood circuit 6 comprises at least a blood pump 17 for controlling and managing the suitable blood flow in the extracorporeal blood circuit 6. Also the blood pump 17 is generally a peristaltic pump.

A concentrate sensor 12 is placed on the blood withdrawal line 6a of the extracorporeal blood circuit 6 between the injection point 9a of the main infusion line 9 and the filtration unit 2 for monitoring the concentration of at least one substance detectable into blood flowing into the blood withdrawal line 6a between an injection point 9a of the main infusion line 9 and the filtration unit 2. In particular, the concentrate sensor 12 is interposed between the blood pump 17 and the injection point 9a of the main infusion line 9, upstream the pressure sensor 48. It is also possible to provide the concentrate sensor 12 between the blood pump 17 and the injection point 9a of the main infusion line downstream from the pressure sensor 48.

Following the direction of blood circulation, a gas exchanger 46 for removing $CO_2$ from circulating blood may be connected to the extracorporeal blood circuit 6. The gas exchanger 46 is in fluid communication with the extracorporeal blood circuit 6 to receive extracorporeal blood, allow $CO_2$ removal from blood and returning blood to the blood circuit at a downstream point. Figure 2 shows a gas exchanger 46 placed upstream the filtration unit 2. However, the gas exchanger may be alternatively positioned downstream the filtration unit 2 along blood circulation direction. Blood circulation direction during normal use of the apparatus is indicated in figure 1 with an arrow 200 which also represent the blood flow rate direction. The gas exchanger 46 is connected in series with the filtration unit 2 and is placed downstream the injection point 9a of the main infusion line 9 where the infusion solution I (regional anticoagulation) is delivered to the blood withdrawal line 6a of the extracorporeal blood circuit 6. The gas exchanger 46 has a blood chamber and a gas chamber separated by a membrane permeable to gases, in particular $CO_2$. The gas exchanger comprises a gas inlet, which may be connected to a gas source, such as the medical gas supply system in a hospital to receive pressurized air or oxygen for example, and a gas outlet in fluid communication with the gas chamber to discharge exhausted gas having removed $CO_2$ from extracorporeal blood circuit 6. The blood inlet and the blood outlet put the extracorporeal blood circuit 6 in fluid communication with the gas exchanger blood chamber. Gas inlet, gas outlet, and gas supply are not shown in figure 2.

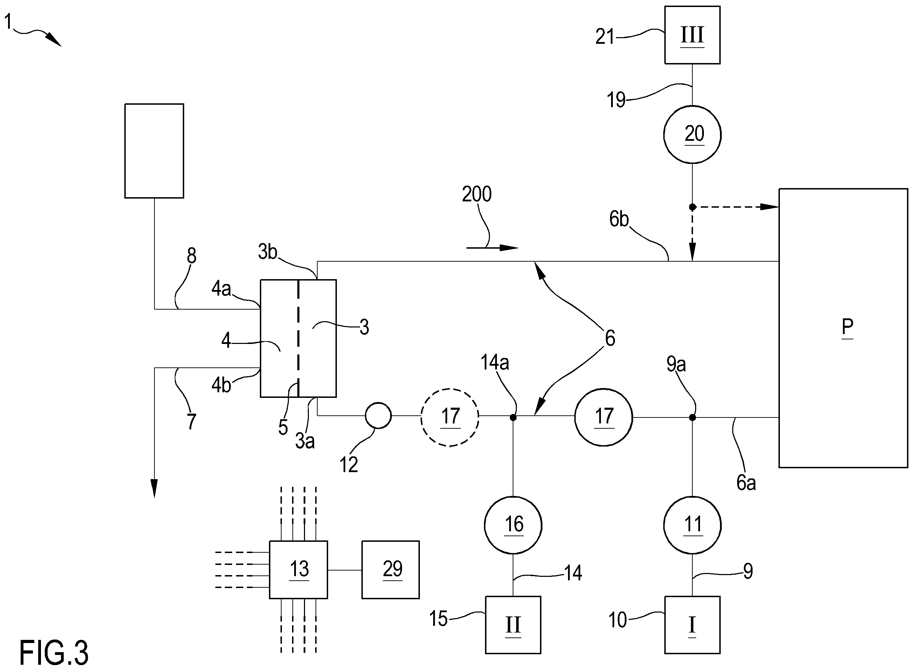

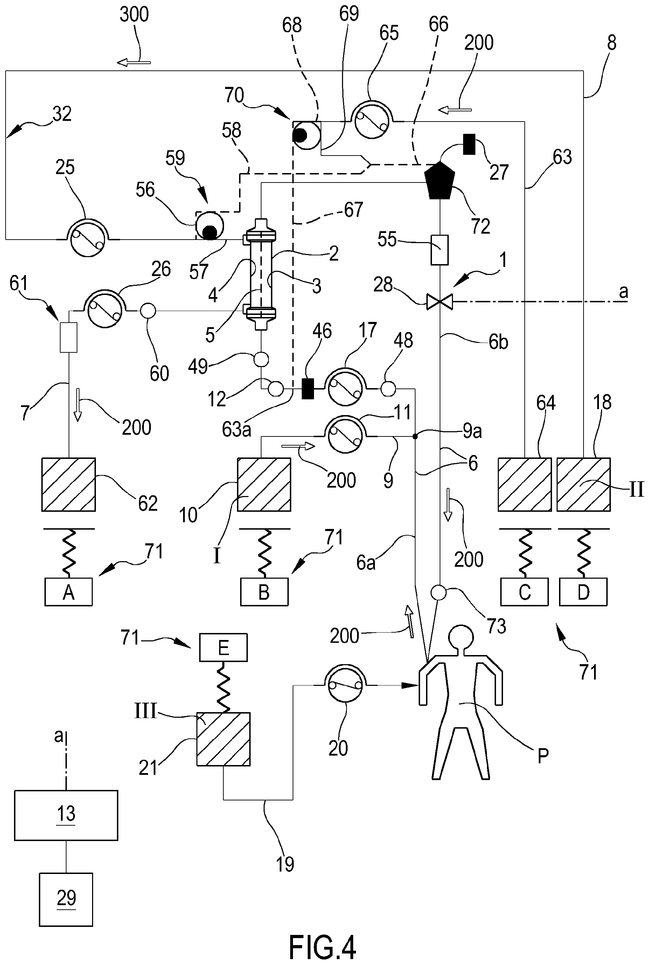

The embodiments represented in figures 2 and 4 show both the gas exchanger 46 and the filtration unit 2. Of course, in some embodiments, only a filtration unit 2 is present with no gas exchanger 46 (see e.g., figure 1 and 3); in other embodiments not represented, only the gas exchanger 46 may be present and no filtration unit 2 is used.

Then, the blood passes through another pressure sensor 49 controlling the correct flow within the blood circuit, which is placed between the blood pump 17 and the filtration unit 2 but it may also interposed between the blood pump 17 and the gas exchanger 46.

After passing through the primary chamber 3 of the filtration unit 2, where the suitable exchanges of substances, molecules and fluids occur through the corresponding semipermeable membrane 5, the treated blood enters the blood return line 6b, first passing through the air separator 72, commonly known as "bubble trap", designed so as to ensure the removal of air bubbles present in the blood. The treated blood getting out of the air separator 72 then passes through an air bubble sensor 55 verifying the absence of said dangerous formations within the treated blood that has to be reintroduced in the patient's blood circulation. Immediately downstream from the bubble sensor 55, the safety valve 20 (or venous clamp) is placed which, in case of alarm, may block the blood flow towards the patient P. A return pressure sensor 73 is also associated to the blood return line 6b in correspondence of the venous access to the patient P.

The return pressure sensor 73 monitors the pressure in the return line and the control unit 13 may use its pressure signal to detect a return line disconnection. In particular, should the bubble sensor 55 detect the presence of air in the blood flow, the machine through safety valve 20 would be able to block immediately the passage of blood so as to avoid any consequence to the patient P. Downstream from the safety valve 20, the treated blood is then carried back to the patient P undergoing therapy. The extracorporeal blood treatment apparatus 1 of figure 2 is equipped with a dialysis fluid circuit 32, which is also provided with at least a dialysis supply line 8 leading into the filtration unit 2 and with a dialysate line 7 from the filtration unit 2. At least a primary fluid container, defining said dialysis liquid source 18, is designed to supply the supply line 8 of the dialysis fluid circuit 32 (generally the primary fluid container shall consist of one or more bags containing a suitable dialysis liquid). The supply line 8 includes means for conveying fluid such as at least a dialysis fluid pump 25 (in the embodiment of figure 2 a peristaltic pump) for controlling the flow rate of the corresponding dialysis liquid from the bag and for defining a direction 300 of dialysis fluid circulation. Downstream from the dialysis fluid pump 25 in the direction of circulation 300 there is a branching 56 splitting the dialysis supply line 8 up into an intake branch 57 and an infusion branch 58. In particular, the infusion branch 58 is connected to the blood return line 6b of the extracorporeal blood circuit 6. In other words, through said infusion branch 58 it is possible to obtain a post-infusion directly in the blood return line 6b using the content of the primary fluid container.

Conversely, the intake branch 57 conveys the fluid directly to the filtration unit 2 and in particular to the secondary chamber 4 of said unit. The dialysis fluid circuit 32 is further equipped with selecting means 59 for determining the percentages of fluid flow within the infusion branch 58 and the intake branch 57. Generally said selecting means 59, usually placed near the branching 56, may be positioned at least between a first operating condition in which they allow the passage of fluid in the intake branch 57 and block the passage in the infusion branch 58, and a second operating condition in which they allow the passage of fluid in the infusion branch 58 and block the passage in the intake branch 57. In other words, said selecting means 59 may consist of a valve element operating on the dialysis fluid circuit 32 by alternatively blocking the passage of fluid in either branch. Suitable selectors may be alternatively provided, which are able to establish a priori the amount of liquid that has to pass through both branches simultaneously. It will also be possible to vary the percentages of fluid in either branch as a function of time and of the pre-established therapies. The dialysis liquid through the intake branch 57 gets into the secondary chamber 4 of the filtration unit 2. In particular, the primary chamber 3 through which the blood flow passes is separated from the secondary chamber 4 through which the dialysis liquid passes through the semi-ipermeable membrane 5 ensuring the suitable passage of the dangerous substances/molecules and of fluid from the blood towards the dialysis liquid mainly by means of convection and diffusion processes, and also ensuring through the same principles the passage of substances/molecules from the dialysis liquid towards the blood. The dialysis fluid then gets into the dialysate line 7 and passes through a suitable dialysate pressure sensor 60. Means is provided for conveying fluid, for instance a dialysate pump 26 controlling the flow rate of the dialysate line 7 within the fluid circuit 32. Also said pump will generally be a peristaltic pump. The fluid to be eliminated then passes through a blood detector 61 and is conveyed into a collection container or bag 62. The hydraulic circuit of the apparatus 1 according to figure 2 includes at least another infusion line 63 for feeding fluid into the blood return line 6b of the extracorporeal blood circuit 6. In particular, the infusion fluid is taken from at least an auxiliary container 64 and is sent directly to the blood return line 7 of the blood circuit 6 through means for conveying fluid, generally an infusion pump 65 (in the example a peristaltic pump) controlling its flow rate. In particular, the infusion liquid may be introduced directly into the air separator 72.

As can also be inferred, the infusion branch 58 of the dialysis fluid circuit 32 and the infusion line 63 are equipped with a common end length 66 letting fluid to enter into the extracorporeal blood circuit 6. Said intake end length 66 is placed downstream from the infusion pump 65 with respect to a direction of infusion and carries the fluid directly into the air separator 72. Further, referring to the diagram in figure 2, the infusion line 63 comprises at least a pre-infusion branch 67 connected to the blood withdrawal line 6a of the blood circuit 17. In further detail, downstream from the infusion pump 65 with respect to the direction of infusion, there is an infusion branching 68 splitting the infusion line 63 up into the pre-infusion branch 67 and post-infusion branch 69. The pre-infusion branch 67, in particular, carries the fluid taken from the bag 64 into the blood withdrawal line 6a of the extracorporeal blood circuit 6 downstream from the blood pump 17 and downstream the gas exchanger 46 with respect to the direction of blood circulation.

Conversely, the post-infusion branch 69 is connected directly to the common end length 66. The infusion line 63 further comprises selecting means 70 for determining the percentage of liquid flow to be sent to the post-infusion branch 69 and to the pre-infusion branch 67. The selecting means 70 placed near the branching 68 may be switched between at least a first operating condition in which they allow the passage of fluid in the pre-infusion branch 67 and block the passage in the post-infusion branch 69, and at least a second operating condition in which they allow the passage of fluid in the post-infusion branch 69 and block the passage in the pre-infusion branch 67. Obviously, as in the case of the selecting means 59 present on the dialysis fluid circuit 32, also the other selecting means 70 will be able to determine the percentage of fluid that has to pass in each of the two branches and to possibly vary it in time in accordance with the planned therapies. Moreover, the selecting means 59 and the other selecting means 70 will generally, though not necessarily, be of the same nature.

The apparatus 1 is equipped with means 71 for determining at least the weight of the primary fluid container defining the dialysis liquid source 18 and/or of the auxiliary fluid container 64 and/or of the container 10 of the infusion solution I (regional anticoagulant), i.e. the infusion fluid source 10 and/or of the collection container 62. In particular, said means 71 comprises weight sensors, for instance respective scales A, B, C, D and E (for example at least an independent sensor for each fluid bag associated to the apparatus 1).

It should then be pointed out that there is a control unit 13 (CPU) active (at least) on the extracorporeal blood circuit 6 and in particular active on the pressure sensor 48 for reading pressure values, on the blood pump 17, on the gas exchanger 46, on the other pressure sensor 49, on the device for detecting the presence of air bubbles 55, on its respective safety valve 20 and on the return pressure sensor 73.

The control unit 13 has also to control the main infusion pump 11 to manage the infusion of the infusion solution I into the blood coming from the patient P and is active on the concentrate sensor 12 for reading the signals emitted by this latter regarding the monitoring of at least one substance S to be detected into the blood infused with the infusion solution I or into blood directly coming from the patient P.

Similarly to the embodiment shown and disclosed with reference to figure 1, the control unit 13 of the embodiment shown in figure 2 works in the same manner.

In particular, the control unit 13 is configured to drive the main infusion pump 11 between the activated condition and the inactivated condition and to receive signals from the concentrate sensor 12 to determine the concentration of the at least one substance in a first (i) and in a second (ii) operating condition of the extracorporeal blood treatment apparatus 1. In the first operating condition (i), the control unit 13 activates the main infusion pump 11 and acquires a plurality of signals from the concentrate sensor 12 to determine the concentration of the at least one substance into blood infused with the infusion solution I. In the second operating condition (ii), the control unit 13 inactivates the main infusion pump 11 and acquires at least one signal from the concentrate sensor 12 to determine the concentration of the same substance into blood when no infusion solution I is infused into the blood. It has to be noted that, in the second operating condition (ii), the control unit 13 is configured to keep the main infusion pump 11 in the inactivated condition to allow blood with no infusion solution I infused by the main infusion line 9 to reach the concentration sensor 12. In this way, the control unit 13 detects the concentration of the substance to be monitored into blood when blood with no infusion solution I reaches the concentration sensor 12. In other words, in the second operating condition (ii) the control unit 13 acquires the concentration measurements, provided by the concentration sensor 12 of the corresponding systemic substance to be detected included into the blood coming from the patient P and not varied by any infusion.

The control unit 13 has also to control the dialysis fluid circuit 32 and, in particular, shall be input with the data detected by the scales A, B, C, D and (possibly) E.

Concerning the weight of the bag constituting the dialysis liquid source 18, the control unit 13 shall control the pump 25, the dialysate pump 26 and the selecting means 59, receive signals from the pressure sensor 60, and shall eventually receive the data detected by the scale A whose function is to determine the weight of the collection container 62.

The control unit 13 shall also control the infusion line 63 checking the weight of the auxiliary container 64 (checked by the scale C) and will be able to control both the infusion pump 65 and the other selecting means 70.

The control unit 12 shall also control the main infusion line 9 through the detection of the weight of the infusion fluid source 10 through the scale B and suitably controlling the infusion pump 11 according to the treatments to be carried out as below detailed and explained.

As apparent, a regional anticoagulation system may be implemented both in the apparatus 1 of figure 2 as well as in any apparatus of the other embodiments (figures 1, 3 and 4), to provide anticoagulation restricted to the extracorporeal blood circuit 6.

The apparatus 1 according to each embodiment shown in figures 1 to 4 may be alternatively (or additionally) provided with a systemic anticoagulation system, such as a syringe pump for injecting heparin downstream the blood pump 17.

Indeed, the algorithm of embodiments the invention as described in the subsequent detailed description works both in CRRT treatment configurations with RCA and in CRRT treatment configurations with systemic (or no) anticoagulation without RCA.

The regional anticoagulation system is described in detail following the description of all the embodiments (figures 1 to 4).

With reference to the embodiment shown in figure 3, the extracorporeal blood treatment apparatus 1 is quite similar to the embodiment shown in figure 1, because it comprises:

- the same main infusion line 9 provided with the same main infusion pump 11 and the same infusion fluid source 10;
- the same blood pump 17 downstream from the injection point 9a of the main infusion line 9;
- the same filtration unit 2 downstream from the blood pump 17;
- the same dialysate line 7 and the same supply line 8 connected to the filtration unit 2; and,

- the same ion replenishment infusion line 19 provided with the same ion replenishment infusion pump 20 and the same source 21 of ion replenishment solution III.

Differently from the apparatus 1 according to the embodiment shown in figure 1 and 2, the concentrate sensor 12 is placed between the blood pump 17 and the filtration unit 2.

In addition to the apparatus according to the embodiment shown in figure 1, the apparatus 1 according to the embodiment shown in figure 3 comprises:

- an auxiliary infusion line 14 connected to the blood withdrawal line 6a of the extracorporeal blood circuit 6 through a corresponding injection point 14a between the injection point 9a of the main infusion line 9 and the concentrate sensor 12;
- an auxiliary fluid source 15 of an auxiliary infusion solution II active on the auxiliary infusion line 14;
- an auxiliary infusion pump 16 active on the auxiliary infusion line 14 and drivable between an activated condition wherein the auxiliary infusion pump 16 causes the auxiliary infusion solution II to flow from the corresponding auxiliary fluid source 15 to the blood withdrawal line 6a of the extracorporeal blood circuit 6, and an inactivated condition, wherein the auxiliary infusion pump 16 is inactive and the auxiliary infusion solution II does not flow through the corresponding auxiliary infusion line 14.

The concentrate sensor 12 is placed on the blood withdrawal line 6a between the auxiliary injection point 14a of the auxiliary infusion line 14 and the filtration unit 2, namely downstream both infusion line injection points.

In addition, the control unit 13 is also operatively connected to the auxiliary infusion pump 16 of the auxiliary infusion line 14 to drive the auxiliary infusion pump 16 between the activated condition and the inactivated condition and respectively determine the concentrate sensor 12 to detect the concentration of at least one substance S into blood flowing into the blood withdrawal line 6a between the auxiliary injection point 14a and the filtration unit 2.

According to the embodiment of the apparatus 1 shown in figure 3, the control unit 13 is configured to receive a plurality of signals from the concentrate sensor 12 to determine the concentration of the at least one substance S in several different working conditions, in particular four working conditions (i), (ii), (iii) and (iv).

In the first working condition (i), the control unit 13 activates the main infusion pump 11, inactivates the auxiliary infusion pump 16 and acquires the signals from the concentrate sensor 12 to determine the concentration of at least one predetermined substance into blood infused only with the infusion solution I.

More in particular, in the first working condition (i), the control unit 13 is configured to keep the main infusion pump 11 in the activated condition and the auxiliary infusion pump 16 in the inactivated condition to allow the concentration sensor 12 to detect at least one predetermined substance S into blood infused with the infusion solution I supplied by the infusion fluid source 10 through the main infusion line 9.

The main infusion pump 11 is kept in the activated condition and the auxiliary infusion pump 16 is kept in the inactivated condition for a time period:

- which is predetermined, or
- which is at least a function of the blood flow rate on the blood withdrawal line 6a of the extracorporeal blood circuit 6; and/or,
- which is at least a function of a volume of a portion of the blood withdrawal line (6a) of the extracorporeal blood circuit (6) between the injection point (9a, 14a) of the main infusion line (9) and/or the auxiliary infusion line (14) and the concentrate sensor (12).
- which allows the concentrate sensor (12) to read a stable concentration value.

In the second working condition (ii), the control unit 13 inactivates the main infusion pump 11, activates the auxiliary infusion pump 16 and acquires the signals from the concentrate sensor 12 to determine the concentration of at least one predetermine substance S into blood infused only with the auxiliary infusion solution II.

In the second working condition (ii), the control unit 13 is configured to keep the main infusion pump 11 in the inactivated condition and the auxiliary infusion pump 16 in the activated condition to allow the concentration sensor 12 to detect at least one substance S into blood infused with the auxiliary infusion solution II supplied by the auxiliary fluid source 16 through the auxiliary infusion line 14.

The main infusion pump 11 is kept in the inactivated condition and the auxiliary infusion pump 16 in kept in the activated condition for a time period:

- which is predetermined, or
- which is at least a function of the blood flow rate on the blood withdrawal line 6a of the extracorporeal blood circuit 6,
- which is at least a function of a volume of a portion of the blood withdrawal line 6a of the extracorporeal blood circuit 6 between the injection point 14a of the auxiliary infusion line 14 and the concentrate sensor 12; and/or,
- which allows the concentrate sensor 12 to read a stable concentration value.

In the third working condition (iii), the control unit 13 activates the main infusion pump 11 and the auxiliary infusion pump 16 and acquires the signal from the concentrate sensor 12 to determine the concentration of the at least one substance S into blood infused with the infusion solution I and the auxiliary infusion solution II.

In particular, the control unit 13 is configured to keep the main infusion pump 11 and the auxiliary infusion pump 16 in the activated condition to allow the concentration sensor 12 to detect at least one substance S into blood infused with the infusion solution I supplied by both the infusion fluid source 10 through the main infusion line 9 and the auxiliary infusion solution II supplied by the auxiliary fluid source 16 through the auxiliary infusion line 14.

The main infusion pump 11 and the auxiliary infusion pump 16 are both kept in the activated condition for a time period:

- which is predetermined; and/or,
- which is at least a function of the blood flow rate on the blood withdrawal line 6a of the extracorporeal blood circuit 6; and/or,
- which is at least a function of a volume of a portion of the blood withdrawal line 6a of the extracorporeal blood circuit 6 between the injection point 9a, 14a of the main infusion line 9 and/or the auxiliary infusion line 14 and the concentrate sensor 12, and/or,
- which allows the concentrate sensor 12 to read a stable concentration value.

In the fourth working condition, the control unit 13 inactivates both the main infusion pump 11 and the auxiliary infusion pump 16 and acquires the signal from the concentrate sensor 12 to determine the concentration of the at least one substance S into blood when no infusion solutions I, II are infused into the blood.

In particular, in the fourth working condition (iv), the control unit 13 is configured to keep the main infusion pump 11 and the auxiliary infusion pump 16 in the inactivated condition to allow blood with no infusion solution I, II infused by the main infusion line 9 and/or by auxiliary infusion line 14 to reach the concentration sensor 12, the control unit 13 detecting the concentration of the at least one substance S into blood when blood with no infusion solution I, II reaches the concentration sensor 12.

The main infusion pump 11 and the auxiliary infusion pump 16 are both kept in the inactivated condition for a time period:

- which is predetermined, or
- which is at least a function of the blood flow rate on the withdrawal line 6a of the extracorporeal blood circuit 6,
- which is at least a function of a volume of a portion of the withdrawal line 6a of the extracorporeal blood circuit 6 between the injection point 9a, 14a of the main infusion line 9 and/or the auxiliary infusion line 14 and the concentrate sensor 12; and/or,
- which allows the concentrate sensor 12 to read a stable concentration value.

The working conditions (i), (ii), (iii), and (iv) according to the embodiment shown in figure 3 may be performed by the control unit 13 according to different sequences or different combinations of different sequences of at least two of the working conditions (i), (ii), (iii) and (iv).

Moreover, at least one sequence of the above mentioned working conditions (i), (ii), (iii), and (iv), optionally each of them, may be repeated by the control unit 13, particularly a plurality of times during an extracorporeal blood treatment.

The control unit 13 is configured to adjust the amount of the auxiliary infusion solution II supplied by the auxiliary fluid source 15 to be infused into the withdrawal line 6a on the base of the concentration of at least one substance detected by the concentrate sensor 12.

Alternatively, the control unit 13 may be configured to adjust the amount of the infusion solution I supplied by the infusion fluid source 10 to be infused into the withdrawal line 6a and the amount of the auxiliary infusion solution II supplied by the auxiliary fluid source 15 to be infused into the withdrawal line 6a on the base of the concentration of at least one substance detected by the concentrate sensor 12.

The concentrate sensor 12 according to the embodiment shown in figure 3 is configured to detect into blood or blood infused with the infusion solution I supplied by the infusion fluid source 10 or blood infused with the auxiliary infusion solution II supplied by the auxiliary fluid source 15 or into blood infused with the infusion solution I supplied by the infusion fluid source 10 and the auxiliary infusion solution II supplied by the auxiliary fluid source 15 at least ionized calcium.

The concentrate sensor 12 may also be configured to detect, in each of the above-mentioned blood/blood mixtures (i.e., patient's blood, blood infused with the infusion solution I, blood infused with the auxiliary infusion solution II and blood infused with the infusion solution I and the auxiliary infusion solution II), the concentration of two (or more) different substances S, wherein one of the two different substances S of interest may be ionized calcium. The detection of the two substances S may be performed separately or simultaneously. It is also possible to provide, as the infusion solution I, at least citrate as anticoagulant.

The auxiliary infusion solution II may comprise a substitute solution, including electrolytes and at least one buffer agent such as bicarbonate.

According to a further alternative that may be seen in figure 3 by a dotted circular line, the blood pump 17 may be placed between an injection point 14a of the auxiliary infusion line (14) and the filtration unit 2. In other words, according to the dotted alternative shown in figure 3, the injection point 14a of the auxiliary infusion line 14 is interposed between the blood pump 17 (depicted by a dotted circular line) and the injection point 9a of the main infusion line 9, being this latter upstream the injection point 14a of the auxiliary infusion line 14 which is upstream the blood pump 17. The alternative dotted blood pump 17 of figure 3, is placed upstream the concentrate sensor 12 which is upstream the filtration unit 2.

With reference to the embodiment shown in figure 4, it has to be noted the apparatus' layout is nearly identical to the one shown in figure 2, except for the fact that the concentrate sensor 12 is placed downstream the blood pump 17 and downstream any injection points 9a, 63a, of the corresponding infusion lines 9, 63, provided in the extracorporeal blood circuit 6 and connected to the corresponding blood withdrawal line 6a of this latter.

Should the extracorporeal blood circuit 6 have more than two infusion lines the corresponding infusion pumps of which are drivable by the control unit 13 to allow the concentrate sensor 12 to detect into the blood infused with the corresponding infusion solutions one or more specific substances, any corresponding injection point has to be placed, along the blood withdrawal line 6a del the extracorporeal blood circuit 6 upstream the concentrate sensor. The activation or the inactivation of each infusion pump of the corresponding infusion line, places upstream the concentration sensor, by the control unit 13, allows the concentrate sensor 12 to be reached by blood not infused, or infused with at least one infusion solution, being able to always detect the concentration of the same substances in different fluids flowing towards the filtration unit 2.

MONITORING METHOD

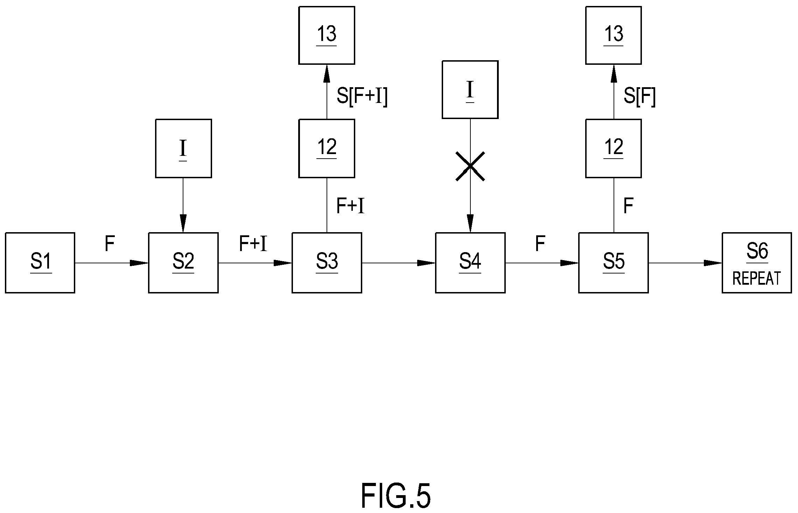

With reference to figure 5, it is schematically represented a monitoring method MM for monitoring at least one substance S into a fluid F, in particular blood, flowing through an extracorporeal blood circuit 6 of an extracorporeal blood apparatus 1 of the type of the one shown in figures 1 to 4 and disclosed above.

As shown in figure 5 the monitoring method MM comprises the steps of: supplying S1 a fluid F to be monitored, in particular blood as withdrawn from a patient P, to the filtration unit 2; infusing S2, upstream the filtration unit 2, at least one infusion solution I into the fluid F supplied to the filtration unit 2; detecting S3 the concentration of at least one substance S into the fluid F infused with at least one of the infusion solution I, downstream the injection point 9a of the main infusion line 9 and upstream the filtration unit 2; interrupting S4 the infusion of the infusion solution I into the fluid F supplied to the filtration unit 2 to allow a fluid F not infused with any infusion solution I to flow towards the filtration unit 2; S5 detecting the concentration of at least one substance S into the fluid F not infused with at least one of the infusion solution I, downstream the injection point 9a of the main infusion line 9 and upstream the filtration unit 2.

Optionally, the monitoring method MM can also comprise a step of repeating S6 one or more steps of S1 to S5, or one or more sequences of the steps of S1 to S5 for a number of times sufficient to monitor the patient and/or the treatment and possibly to outline a trend over time.

In order to perform the steps of detecting S3 and S5, a control unit 13 configured to drive the main infusion pump 11 between the activated and the inactivated condition is provided. In this manner, the infusion solution I supplied by the fluid source 10 is allowed or not to flow into the blood advancing towards the filtration unit 2. The infused or not infused blood reaches the concentrate sensor 12 before arriving at the filtration unit 2 placing the concentrate sensor 12 in the condition of detect the at least one substance S into the blood, infused or not, passing at that time.

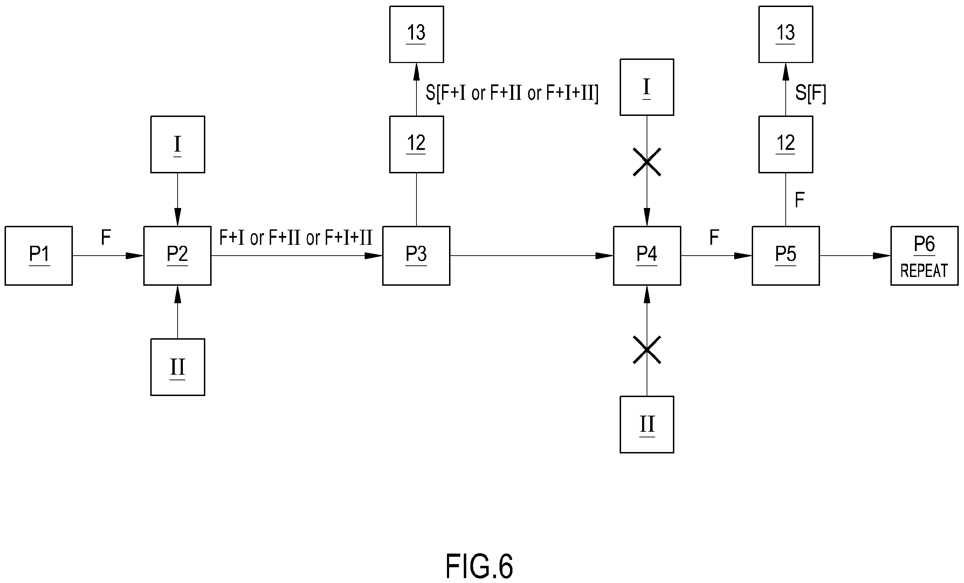

The monitoring method MM sketched in figure 5 may be roughly traced back to the apparatus shown figures 1 and 2, whereas the monitoring method MM sketched in figure 6 may be roughly traced back to the apparatus shown figures 3 and 4.

With reference to figure 6, the monitoring method MM comprises the steps of: supplying P1 a fluid F to be monitored, in particular blood as withdrawn from a patient P, to the filtration unit 2; infusing P2, upstream the filtration unit 2, at least one infusion solution I and/or at least one auxiliary infusion solution II into the fluid supplied to the filtration unit 2; detecting P3 the concentration of at least one substance S into the fluid F infused with at least one of the infusion solutions I, II downstream the corresponding injection point 9a, 14a of the corresponding infusion line 9, 14 and upstream the filtration unit 2; interrupting P4 the infusion of at least one of the infusion solutions I, II into the fluid supplied to the filtration unit 2 (not represented in figure 6) or interrupting the infusion of both infusion solutions I, II (as shown in figure 6) into the fluid supplied to the filtration unit 2 to respectively allow a fluid F infused with only one infusion solution I, II or a fluid F not infused with any infusion solution I, II, to flow towards the filtration unit (2); detecting P5 the concentration of at least one substance into the fluid F infused with only one infusion solution I, II or not infused with any infusion solution I, II downstream the corresponding injection point 9a, 14a of the corresponding infusion line 9, 15 and upstream the filtration unit 2.

Optionally, the monitoring method MM may also comprise a step of repeating P6 one or more steps of (P1) to (P5), or one or more sequences of the steps of (P1) to (P5) for a number of times sufficient to monitor the patient and/or the treatment and possibly to outline a trend over time.

It has to be noted that in order to perform the steps of detecting P3 and P5, it is necessary to provide a control unit 13 able to drive the main infusion pump 11 and/or the auxiliary infusion pump 16 between the activated and the inactivated condition. In this manner, the infusion solutions I, II supplied by the fluid source 10 and the auxiliary fluid source 15 are allowed or not to flow into the blood advancing towards the filtration unit 2. The infused or not infused blood reaches the concentrate sensor 12 before arriving at the filtration unit 2 placing the concentrate sensor 12 in the condition of detecting the at least one substance S into the blood, infused or not, passing at that time.

THE REGIONAL ANTICOAGULATION SYSTEM

As previously discussed embodiments of the present description may be used in the context of regional anticoagulation. As below further explained, when the invention is applied to a therapy including regional anticoagulation, the infusion fluid source 10 connected to the main infusion line 6a previously described contains a regional anticoagulant (citrate). Correspondingly, a further ion replacement infusion of concentrated calcium (and possibly also magnesium) may be present to infuse the ion replacement solution either into the blood return line 6b or directly into the patient in order to balance the ions loss. In this embodiment, during the first operating condition (steps S1-S3; steps P1-P3), the concentrate sensor 12 is used to measure (at least) the ionized calcium concentration after citrate infusion (causing an ion calcium concentration reduction due to complexing with citrate). Further, in the second operating condition (i.e. when the citrate infusion is stopped - steps S4-S5; steps P4-P5), the concentrate sensor 12 is used to measure (at least) patient systemic ionized calcium.

The regional anticoagulation system comprises the source of regional anticoagulant 10, e.g., a container or a bag containing at least a substance having an anticoagulant effect. For example, citrate, in the form of pure sodium citrate ($Na_3$citrate) or mixture of sodium citrate and citric acid are used for blood anticoagulation purpose. Alternatively pure citric acid may be used as anticoagulant. Indeed, citrate has a high affinity for calcium in creating complexes and several steps of the coagulation cascade are dependent on blood (ionized) calcium. A proper decrease of ionized calcium concentration in the presence of citrate inactivates the coagulation cascade. Normal plasma includes about 1.1 to 1.3 mmol/l of ionized calcium, 0.1 -0.2 mmol/l of complexed calcium and 0.9 to 1.2 mmol/l of protein-bound calcium. In order to achieve proper anticoagulation effects, general guidelines are to adjust citrate amount/dose as to reach an ionized calcium concentration of 0.20 to 0.35 mmol/l in the extracorporeal blood circuit after citrate infusion. Plasma with citrate addition for anticoagulation purposes would include (as an average) about 0.3 mmol/l of ionized calcium, 1.8 mmol/l of complexed calcium (mainly $Ca_3$citrate$_2$) and 0.2 mmol/l of protein-bound calcium. During RCA, intensity of anticoagulation may be adjusted via the amount of infused citrate. Measurements from concentrate sensor 12 allows to provide ionized calcium concentration values providing an indication to the physician about the achievement of the proper anticoagulation effects. If the measured values lie outside specified ranges (see previous passages in the aspects and in the previous description), correction may be implemented by increasing or reducing the citrate infusion depending on the actual ionized calcium concentration detected during active citrate infusion.

Post-filtration unit ionized calcium concentration is commonly used as key parameter (target in the 0.20-0.35 mmol/l range); however the ionized calcium concentration directly upstream the dialyzer may also be used as a proper indicator (with or without any correction to the measured value to take into account of the effect of filtration in the dialyzer). The regional anticoagulation system is arranged to deliver the regional anticoagulant at a delivery point that coincides with the injection point 9a of the main infusion line 9 in the extracorporeal blood circuit 6. Citrate infusion is in particular administered close to an access end of the blood withdrawal line 6a to get full anticoagulation of the extracorporeal blood circuit 6. In general, the delivery point of the regional anticoagulant is located upstream the blood pump 17 and, in the embodiments shown in figures 1 to 4 may coincide with the injection point 9a of the main infusion line 9.

When citrate is infused into the blood withdrawal line 6 close to the patient vascular access, blood pump speed is automatically adjusted as to take the operator set blood flow rate from access site (blood pump speed=k*($Q_b$+$Q_{cit}$), wherein $Q_b$ is the set blood flow rate - desired at the access site and Qcit is the citrate infusion flow rate).

Citrate amount is prescribed through the 'Citrate Dose' parameter (Dcitrate) which is the amount of citrate per liter of blood treated (mmol/l blood). Notably, citrate dose does not match with citrate concentration in the diluted blood reaching the filtration unit. The concept is rather to provide for an amount of citrate in proportion to the amount of calcium to be chelated. The set of the citrate pump 11 is:

$$Qcit = \frac{Dcitrate}{[citrate]_{PBP}} \times Qb$$

wherein:

$Q_{cit}$ is the citrate infusion flow rate;
$Q_b$ is the set blood flow rate;
$D_{citrate}$ the citrate dose; and
$[citrate]_{PBP}$ is the citrate concentration in the anticoagulant source.

Citrate infusion is delivered with a dosage aimed to maintain ionized calcium level around 0.25- 0.35 mmol/l in the blood circuit downstream the dialyzer. Typically, citrate dose is included in the range 1.5 to 6.0 mmol/L-of-blood. The most common range is 2.5 to 4.0 mmol/L-of-blood. Citrate dose guideline of 3.0 mmol/L-of-blood is globally followed.

Ionized calcium and citrate complexes are rather small molecules which are easily transferred through the filtration unit 2. Loss rates are basically dependent on flow rates, filter efficiency with respect to small molecules and solute concentrations. While about half of the total calcium is not available to mass transfer during standard anticoagulation (since it is protein-bound), about 90% of total calcium becomes available during citrate anticoagulation. Therefore, citrate regional anticoagulation combined with the use of calcium free dialysis and/or replacement fluids implies significant calcium losses to dialysate. In extracorporeal blood treatments with RCA, calcium infusion is required to balance calcium losses to dialysate. During RCA, calcium infusion is adjusted to keep patient systemic ionized calcium in the normal range (e.g., 1.0 - 1.2 mmol/l). Therefore, the regional anticoagulation system of the apparatus 1 includes a source of ion balancing solution 21, which is reinfused in the blood, either in the return line 7, in particular close to the venous vascular access, or directly into the patient P (infusion into central catheter, which is recommended). The ion balancing solution 21, e.g., a syringe, a container, or a bag, comprises a ion replacement infusion line 19 and a corresponding ion replacement pump 20 to drive delivery of a proper ion replacement infusion rate Qca. Figures 1 to 4 show a line 19 that may directly infuse into the patient P. Of course, line 19 may alternatively directly infuse in the blood return line 6b (e.g., figures 1 and 3), possibly close to the venous access. Notably, the auxiliary container 64 of figures 2 and 4 may (alternatively or in combination) be used for ion balancing in the blood return line 6b. In the latter example, pre-infusion branch 67 remains closed and unused to avoid calcium infusion upstream the filtration unit 2. In the example of the attached figures, the syringe pump (not shown) usually used to deliver heparin may be alternatively used to deliver the ion balancing solution either directly into the patient P or alternatively in the blood return line 6b. The ion balancing solution includes ionized (concentrated) calcium and its infusion is performed to restore patient systemic ionized calcium at normal level. Notably, ion balancing solution may include also ionized magnesium and its infusion is performed to restore patient systemic ionized magnesium at normal level since also magnesium removal in dialysate is increased during RCA. The ion replacement infusion rate $Q_{ca}$ may be adjusted based on the revealed patient ionized calcium concentration in blood or an automatic control may be implemented, such as the one described in patent publication US8668825B2.

In an implementation, the ion balancing solution flow rate is kept proportional to the estimated calcium loss rate in dialysate. For example it is computed by the apparatus control unit through the equation:

$$Q_{ca} = \frac{CaComp \cdot J_{ca}}{[Ca]} - \frac{Q_{rep} \cdot [[Ca]_{rep}}{[Ca]}$$

Where CaComp is a calcium compensation parameter, $Q_{ca}$ is the ion balancing solution flow rate (ml/h), Jca is the estimated calcium loss rate in the dialysate (mmol/h), [Ca] is calcium concentration of the ion balancing solution (mmol/l), $Q_{rep}$ is the replacement flow rate (ml/h), and $[Ca_{rep}]$ is calcium concentration of the replacement solution in post-dilution (mmol/l). Calcium compensation is the user-controllable setting, which might be set by the operator generally in a range between 5% and 200%. Notably the above equation takes into account a post replacement solution including calcium. In case no calcium is in the post replacement solution (or no replacement solution is used) the second term of the equation should be disregarded (equal to zero).

Indeed, as to dialysis fluid (and replacement solution), they are generally calcium free to prevent transferring ionized calcium to blood. Moreover, the dialysis and/or replacement fluids have adapted buffer content due to citrate metabolism and adapted sodium if concentrated citrate solution (hypertonic) is used.

The dialysis fluid may contain no buffer agent, e.g., no bicarbonate. A buffer agent from a source/container/bag 64 may be infused into the blood return line 6b via a suitable buffer agent supply line 63, 69, 66 and the corresponding buffer agent pump 65.

## Claims

1.   An extracorporeal blood treatment apparatus (1) comprising:

a filtration unit (2);
an extracorporeal blood circuit (6) comprising a blood withdrawal line (6a) connected to an inlet (3a) of the filtration unit (2), and a blood return line (6b) connected to an outlet (3b) of the filtration unit (2);
at least one main infusion line (9) connected to the blood withdrawal line (6a) of the extracorporeal blood circuit (6) to transfer an infusion solution (I) into blood;
at least one infusion fluid source (10) of the infusion solution (I) connected to the main infusion line (6a);
a main infusion pump (11) active on the main infusion line (9) drivable between an activated condition wherein the main infusion pump (11) causes the infusion solution (I) to flow from the corresponding infusion fluid source (10) to the blood withdrawal line (6a) of the extracorporeal blood circuit (6), and an inactivated condition, wherein the main infusion pump (11) is inactive and the infusion solution (I) does not flow through the corresponding main infusion line (9);
a concentrate sensor (12) placed on the blood withdrawal line (6a) between an injection point (9a) of the main infusion line (9) and the filtration unit (2) for monitoring the concentration of at least one substance detectable into blood flowing into the blood withdrawal line (6a) between the injection point (9a) and the filtration unit (2);
a control unit (13) operatively connected to the concentration sensor (12) and the main infusion pump (11) of the main infusion line (9), the control unit (13) being configured to drive the main infusion pump (11) between the activated condition and the inactivated condition and to receive signals from the concentrate sensor (12) to determine the concentration of the at least one substance in a first and in a second operating condition (ii) of the extracorporeal blood treatment apparatus (1), wherein the control unit (13) is further configured to:

> (i) in the first operating condition - activate the main infusion pump (11) and acquire the signal from the concentrate sensor (12) to determine the concentration of the at least one substance into blood infused with the infusion solution (I); and,
> (ii) in the second operating condition - inactivate the main infusion pump (11) and acquire the signal from the concentrate sensor (12) to determine the concentration of the at least one substance into blood when no infusion solution (I) is infused into the blood.

**2.** An extracorporeal blood treatment apparatus according to the previous claim, wherein the control unit (13) is configured to switch between first and second operating conditions (i), (ii) and to repeat such operating conditions (i) and (ii) a plurality of times during an extracorporeal blood treatment, wherein in the second operating condition (ii), the control unit (13) is configured to keep the main infusion pump (11) in the inactivated condition to allow blood with no infusion solution (I) infused by the main infusion line (9) to reach the concentration sensor (12), the control unit (13) detecting the concentration of the at least one substance into blood when blood with no infusion solution (I) reaches the concentration sensor (12).

**3.** An extracorporeal blood treatment apparatus according to any one of the previous claims, wherein the filtration unit (2) has a primary chamber (3) and a secondary chamber (4) separated by a semi-permeable membrane (5), the blood withdrawal line (6a) being connected to the inlet (3a) of the primary chamber (3) of the filtration unit (2), the blood return line (6b) being connected to the outlet (3b) of the primary chamber (3) of the filtration unit (2), optionally the apparatus comprising a dialysate line (7) connected to an outlet (4b) of the secondary chamber (4) and in particular a supply line (8) connected to an inlet (4a) of the secondary chamber (4).

**4.** An extracorporeal blood treatment apparatus (1) according to any one of the previous claims, wherein in the second operating condition (ii), the control unit

(13) is configured to keep the main infusion pump (11) in the inactivated condition for a time period which is a function of one or more of:

> a blood flow rate in the blood withdrawal line (6a) of the extracorporeal blood circuit (6), in particular in correspondence of the concentrate sensor (12);
> a cross section of the withdrawal line (6a) of the extracorporeal blood circuit (6), in particular at a portion of the withdrawal line (6a) where the concentrate sensor (12) is active;
> a distance on the withdrawal line (6a) of the extracorporeal blood circuit (6) between the injection point (9a) of the main infusion line (9) and the concentrate sensor (12);
> a design of a portion of the withdrawal line (6a) of the extracorporeal blood circuit (6) between the injection point (9a) of the main infusion line (9) and the concentrate sensor (12);
> a volume of a portion of the withdrawal line (6a) of the extracorporeal blood circuit (6) between the injection point (9a) of the main infusion line (9) and the concentrate sensor (12); and/or,
> a time period that allows the concentrate sensor (12) to read a stable concentration value.

**5.** An extracorporeal blood treatment apparatus (1) according to any one of the previous claims, in the first operating condition (i), the control unit (13) is configured to keep the main infusion pump (11) in the activated condition to allow the concentration sensor (12) to detect the at least one substance into blood infused with the infusion solution (I) supplied by the infusion fluid source (10) through the main infusion line (9), in particular the control unit (13) is configured to keep the main infusion pump (11) in the activated condition for substantially the entire treatment except when concentration of the at least one substance into blood when no infusion solution (I) is infused is requested.

**6.** An extracorporeal blood treatment apparatus (1) according to any one of the previous claims, wherein in the second operating condition (ii), the control unit (13) is configured to keep the main infusion pump (11) in the inactivated condition for a time period (t) function of the blood flow rate ($Q_b$) and the volume (V) of a circuit section between the infusion site (9a) and the sensor (12) according to the following relation:

$$t = \alpha \cdot \frac{V}{Q_b} + T_{rsp}$$

wherein:

t = time period;

a = constant greater than 1;

V = volume of the circuit section between the infusion site (9a) and the sensor (12);

$Q_b$ = blood flow rate;

$T_{rep}$ = sensor response time;

wherein in particular sensor response time is zero when concentrate sensor 12 implementing blood sampling is used.

7.  An extracorporeal blood treatment apparatus (1) according to any one of the previous claims, wherein the at least one substance the concentrate sensor (12) is configured to detect into blood or blood infused with the infusion solution (I) supplied by the infusion fluid source (10) is

    • a blood electrolyte such as sodium, potassium, phosphates, magnesium, chloride; and/or
    • hematocrit; and/or
    • hemoglobin; and/or;
    • urea; and/or
    • creatinine; and/or
    • ionized calcium.

8.  An extracorporeal blood treatment apparatus (1) according to any one of the previous claims, wherein the concentrate sensor (12) is configured to detect the concentration of two different substances into blood or blood infused with the infusion solution (I) supplied by the infusion fluid source (10), either

    separately detect the concentration of two different substances in sequence both into blood and into blood infused with the infusion solution (I) supplied by the infusion fluid source (10); or, simultaneously detect the concentration of two different substances into blood or blood infused with the infusion solution (I) supplied by the infusion fluid source (10),

    wherein in particular one of the two different substances is ionized calcium.

9.  An extracorporeal blood treatment apparatus (1) according to any one of the previous claims, wherein the infusion solution (I) supplied by the infusion fluid source (10) includes at least one solute that reacts with a component present in the blood, such as citrate reacting with calcium that is present in the blood and wherein, in the first operating condition (i), the concentrate sensor (12) is configured to measure a reduction of the component that reacted with the solute in the infusion solution (I), in particular the concentration of ionized calcium after complexing with citrate and, in the second operating condition (ii), the concentrate sensor (12) is configured to measure the patient systemic concentration of the at least one substance, in particular the patient systemic concentration of ionized calcium.

10. An extracorporeal blood treatment apparatus (1) according to any one of the previous claims, wherein the control unit (13) is configured to adjust the amount of the infusion solution (I) supplied by the infusion fluid source (10) to be infused into the withdrawal line (6a) on the base of the concentration of the at least one substance detected by the concentrate sensor (12), in particular based on the concentration measured during the first operating condition (i).

11. An extracorporeal blood treatment apparatus (1) according to any one of the previous claims, wherein the extracorporeal blood treatment apparatus (1) comprises only one concentrate sensor (12) active on the extracorporeal blood circuit (6), configured to detect at least one substance into blood or blood infused with the infusion solution (I) supplied by the infusion fluid source (10), in particular configured to detect at least ionized calcium into blood or blood infused with the infusion solution (I) supplied by the infusion fluid source (10), optionally the extracorporeal blood treatment apparatus (1) does not comprise other concentrate sensors except the one active on the blood withdrawal line (6a) of the extracorporeal blood circuit (6) configured to detect at least one substance into blood or blood infused with the infusion solution (I) supplied by the infusion fluid source (10), in particular configured to detect at least ionized calcium into blood or blood infused with the infusion solution (I) supplied by the infusion fluid source (10).

12. An extracorporeal blood treatment apparatus (1) according to any one of the previous claims, wherein the extracorporeal blood treatment apparatus (1) comprises a blood pump (17) active on the extracorporeal blood circuit (6), optionally placed on the withdrawal line (6a) of the extracorporeal blood circuit (6), in particular placed between the injection point (9a) of the main infusion line (9) and the filtration unit (2), in particular placed between the concentrate sensor (12) and the filtration unit (2).

13. An extracorporeal blood treatment apparatus (1) according to any one of the previous claims, wherein the extracorporeal blood treatment apparatus (1) further comprises:

    an ion replenishment infusion line (19);
    an ion replenishment infusion pump (20) active on the ion replenishment infusion line (19); and,
    a source (21) of ion replenishment solution (III) connected to one end of the ion replenishment infusion line (19), the ion replenishment infusion line (19) infusing the ion replenishment solution (19) either into the blood return line (6b) or directly into the patient (P), in particular the ion replenishment solution (III) comprising ionized calcium, specifically a concentrated solution of ionized calcium.

14. An extracorporeal blood treatment apparatus (1) according to any one of the previous claims, wherein the control unit (13) is configured to adjust the amount of the ion replenishment solution (III) supplied by the source (21) of ion replenishment solution (III) on the base of the concentration of the at least one substance detected by the concentrate sensor (12), in particular based on the concentration measured during the second operating condition (ii), such as the patient systemic concentration of ionized calcium measured during the second operating condition (ii).

15. An extracorporeal blood treatment apparatus (1) according to any one of the previous claims, wherein the control unit (13) is configured to:

in the first operating condition (i), compare the measured concentration of the at least one substance with a first high threshold and to issue an alarm in case the measured concentration exceeds the first high threshold, in particular the alarm being issued if ionized calcium concentration is over the first high threshold, such as ionized calcium higher than 0.40 mM; and/or, in the first operating condition (i), compare the measured concentration of the at least one substance with a first low threshold and to issue an alarm in case the measured concentration exceeds the first low threshold, in particular the alarm being issued if ionized calcium concentration is below the first low threshold, such as ionized calcium lower than 0.25 mM; and/or in the second operating condition (ii), compare the measured concentration of the at least one substance with a second low threshold and to issue an alarm in case the measured concentration exceeds the second low threshold, in particular the alarm being issued if ionized calcium concentration is below the second low threshold, such as ionized calcium lower than 0.90 mM; and/or, in the second operating condition (ii), compare configured to compare the measured concentration of the at least one substance with a second high threshold and to issue an alarm in case the measured concentration exceeds the second high threshold, in particular the alarm being issued if ionized calcium concentration is beyond the second high threshold, such as ionized calcium higher than 1.25 mM; and/orin the second operating condition (ii), determine a variation of the measured concentration of the at least one substance and to compare the variation with a drift threshold and to issue an alarm in case the variation exceeds the drift threshold, in particular the alarm being issued if ionized calcium concentration is varying faster than the drift threshold, such as ionized calcium concentration varying, e.g., decreasing and/or increasing, faster than 0.05 mM/h; and/or in the second operating condition (ii), determine a variation of the measured concentration of the at least one substance and to issue an alarm in case an alarm criteria is matched based on both (a) the measured value of concentration of the at least one substance in the second operating condition, and (b) the variation of the measured concentration of the at least one substance in the second operating condition.

FIG.1

FIG.2

FIG.3

FIG.4

S1

F

S2

I

F+I

S3

12

S[F+I]

13

F+I

S4

I

X

F

S5

12

S[F]

13

F

S6

REPEAT

FIG.5

FIG.6

Europäisches
Patentamt

European
Patent Office

Office européen
des brevets

# EUROPEAN SEARCH REPORT

**Application Number**

EP 22 20 3338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/208105 A1 (BRANDL MARTIN [AT] ET AL) 25 August 2011 (2011-08-25) * References 205a,204,205,205b,215,220,22,224; paragraph [0019]; figure 2 * | 1-15 | INV. A61M1/34 A61M1/36 |
| A | US 2005/006296 A1 (SULLIVAN THOMAS A [US] ET AL) 13 January 2005 (2005-01-13) * refrences 254; figure 8.9 * | 1-15 | |

**TECHNICAL FIELDS SEARCHED    (IPC)**

A61M

The present search report has been drawn up for all claims

| Place of search | Date of completion of the search | Examiner |
|---|---|---|
| The Hague | 14 April 2023 | Vadot, Pierre |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 20 3338

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-2023

| Patent document cited in search report | | Publication date | Patent family member(s) | | Publication date |
|---|---|---|---|---|---|
| US 2011208105 | A1 | 25-08-2011 | AT | 505690 A1 | 15-03-2009 |
| | | | EP | 2200675 A1 | 30-06-2010 |
| | | | US | 2011208105 A1 | 25-08-2011 |
| | | | WO | 2009026603 A1 | 05-03-2009 |
| US 2005006296 | A1 | 13-01-2005 | US | 2005006296 A1 | 13-01-2005 |
| | | | WO | 2004105589 A2 | 09-12-2004 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011208105 A
- DE 10114283 C2
- US 2018303995 A
- US 2007007184 A1
- US 8668825 B2